(19) Europäisches Patentamt
European Patent Office
Office européen des brevets

(11) EP 2 824 180 B1

(12) **EUROPEAN PATENT SPECIFICATION**

(45) Date of publication and mention of the grant of the patent:
**03.04.2019 Bulletin 2019/14**

(21) Application number: **12870538.1**

(22) Date of filing: **15.11.2012**

(51) Int Cl.:
***C12Q 1/6837*** *(2018.01)* ***C12Q 1/689*** *(2018.01)*

(86) International application number:
**PCT/JP2012/079691**

(87) International publication number:
**WO 2013/132700 (12.09.2013 Gazette 2013/37)**

(54) **METHOD FOR DETECTING TARGET NUCLEIC ACID**

VERFAHREN ZUR DETEKTION EINER ZIELNUCLEINSÄURE

PROCÉDÉ DE DÉTECTION D'UN ACIDE NUCLÉIQUE CIBLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.03.2012 US 201261606548 P**
**18.10.2012 JP 2012231071**

(43) Date of publication of application:
**14.01.2015 Bulletin 2015/03**

(73) Proprietor: **NGK Insulators, Ltd.**
**Nagoya-shi, Aichi 467-8530 (JP)**

(72) Inventors:
- **NIWA Kousuke**
**Nagoya-shi**
**Aichi 467-8530 (JP)**
- **HIROTA Toshikazu**
**Nagoya-shi**
**Aichi 467-8530 (JP)**

(74) Representative: **Mewburn Ellis LLP**
**City Tower**
**40 Basinghall Street**
**London EC2V 5DE (GB)**

(56) References cited:

| | |
|---|---|
| **WO-A1-03/102228** | **WO-A1-2011/004896** |
| **WO-A1-2011/052586** | **WO-A1-2011/052586** |
| **WO-A2-2007/120843** | **WO-A2-2008/080029** |
| **JP-A- H09 507 024** | **JP-A- 2008 048 705** |
| **JP-A- 2008 306 941** | **JP-A- 2008 504 842** |
| **JP-A- 2009 000 024** | **JP-A- 2009 232 778** |
| **JP-A- 2010 035 451** | **US-A1- 2002 177 141** |

- **NISHIDA ET AL: "Further development of multiplex single nucleotide polymorphism typing method, the DigiTag2 assay", ANALYTICAL BIOCHEMISTRY, ACADEMIC PRESS INC, NEW YORK, vol. 364, no. 1, 31 March 2007 (2007-03-31), pages 78-85, XP022005967, ISSN: 0003-2697, DOI: 10.1016/J.AB.2007.02.005**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 824 180 B1

[Sequence Listings]

SEQUENCE LISTING

<110> NGK INSULATORS, LTD.

<120> Method of Detecting Target Nucleotide

<130> K12-129-PCT

<160> 100

<170> PatentIn version 3.3

<210> 1
<211> 23
<212> DNA
<213> Artificial

<220>
<223> probe

<400> 1
tgttctctga ccaatgaatc tgc 23

<210> 2
<211> 23
<212> DNA
<213> Artificial

<220>
<223> probe

<400> 2
tggaactggg aacgctttag atg 23

<210> 3
<211> 23
<212> DNA
<213> Artificial

<220>

<223> probe

<400> 3
ttcgcttcgt tgtaatttcg gac 23

<210> 4
<211> 23
<212> DNA
<213> Artificial

<220>
<223> probe

<400> 4
aggcatccta agaaatcgct act 23

<210> 5
<211> 23
<212> DNA
<213> Artificial

<220>
<223> probe

<400> 5
tagcccagtg atttatgaca tgc 23

<210> 6
<211> 23
<212> DNA
<213> Artificial

<220>
<223> probe

<400> 6
cgctctggtt actattggac gtt 23

<210> 7
<211> 23
<212> DNA
<213> Artificial

<220>
<223> probe

<400> 7
tagccaactc taaataacgg acg 23

<210> 8
<211> 23
<212> DNA
<213> Artificial

<220>
<223> probe

<400> 8

ttcggttgtc gatatgagga tct 23

<210> 9
<211> 23
<212> DNA
<213> Artificial

<220>
<223> probe

<400> 9
gggggtact tcatacaaga tgc 23

<210> 10
<211> 23
<212> DNA
<213> Artificial

<220>
<223> probe

<400> 10
gagtagcagg caaataccct aga 23

<210> 11
<211> 23
<212> DNA
<213> Artificial

<220>
<223> probe

<400> 11
gcctattaag gtctacgtca tcg 23

<210> 12
<211> 23
<212> DNA
<213> Artificial

<220>
<223> probe

<400> 12
agtcatacag tgaggaccaa atg 23

<210> 13
<211> 23
<212> DNA
<213> Artificial

<220>
<223> probe

<400> 13
cattcgacat aagctgttga tgc 23

<210> 14

<211> 23
<212> DNA
<213> Artificial

<220>
<223> probe

<400> 14
tgctcactta cattacgtcc atg 23

<210> 15
<211> 23
<212> DNA
<213> Artificial

<220>
<223> probe

<400> 15
tacacctatc aactcgtaga gca 23

<210> 16
<211> 23
<212> DNA
<213> Artificial

<220>
<223> probe

<400> 16
aggtccggta gtaatttagg tgc 23

<210> 17
<211> 23
<212> DNA
<213> Artificial

<220>
<223> probe

<400> 17
tgcactctga tatatacagg cca 23

<210> 18
<211> 23
<212> DNA
<213> Artificial

<220>
<223> probe

<400> 18
gcagccctta tagataacgg gac 23

<210> 19
<211> 23
<212> DNA
<213> Artificial

<220>
<223> probe

<400> 19
gaagccatga tactgttcag ggt 23

<210> 20
<211> 23
<212> DNA
<213> Artificial

<220>
<223> probe

<400> 20
tattctacca acgacatcac tgc 23

<210> 21
<211> 23
<212> DNA
<213> Artificial

<220>
<223> probe

<400> 21
ccatcagtta ttcggaggga ctc 23

<210> 22
<211> 23
<212> DNA
<213> Artificial

<220>
<223> probe

<400> 22
ccatatccga ttattagcga cgg 23

<210> 23
<211> 23
<212> DNA
<213> Artificial

<220>
<223> probe

<400> 23
catctccaag aattgaccca cca 23

<210> 24
<211> 23
<212> DNA
<213> Artificial

<220>
<223> probe

<400> 24
ccgtcgtgtt attaaagacc cct 23

<210> 25
<211> 23
<212> DNA
<213> Artificial

<220>
<223> probe

<400> 25
gaaggatcgc ttttatctgg cat 23

<210> 26
<211> 23
<212> DNA
<213> Artificial

<220>
<223> probe

<400> 26
catttgtcag gtacagtcca ctt 23

<210> 27
<211> 23
<212> DNA
<213> Artificial

<220>
<223> probe

<400> 27
gcccacactc ttacttatcg act 23

<210> 28
<211> 23
<212> DNA
<213> Artificial

<220>
<223> probe

<400> 28
cgctgttact gtaagcgtac tag 23

<210> 29
<211> 23
<212> DNA
<213> Artificial

<220>
<223> probe

<400> 29
cgcgattcct attgattgat ccc 23

<210> 30
<211> 23
<212> DNA
<213> Artificial

<220>
<223> probe

<400> 30
ccgtctgggt taaagattgc tag 23

<210> 31
<211> 23
<212> DNA
<213> Artificial

<220>
<223> probe

<400> 31
agtcagtcca aatctcagga tgg 23

<210> 32
<211> 23
<212> DNA
<213> Artificial

<220>
<223> probe

<400> 32
cgcctaaatg aaactcactc tgc 23

<210> 33
<211> 23
<212> DNA
<213> Artificial

<220>
<223> probe

<400> 33
ggggtcaaac caacaattga tct 23

<210> 34
<211> 23
<212> DNA
<213> Artificial

<220>
<223> probe

<400> 34
gcccattgat agaattacga ggc 23

<210> 35
<211> 23
<212> DNA

<213> Artificial

<220>
<223> probe

<400> 35
atgccgttgt caagagttat ggt 23

<210> 36
<211> 23
<212> DNA
<213> Artificial

<220>
<223> probe

<400> 36
tgccggctat cgtaagtata tgc 23

<210> 37
<211> 23
<212> DNA
<213> Artificial

<220>
<223> probe

<400> 37
gcacctcata ccttcataga gca 23

<210> 38
<211> 23
<212> DNA
<213> Artificial

<220>
<223> probe

<400> 38
cgcgacattt agtccaggag atg 23

<210> 39
<211> 23
<212> DNA
<213> Artificial

<220>
<223> probe

<400> 39
ctagtccatt gtaacgaagg cca 23

<210> 40
<211> 23
<212> DNA
<213> Artificial

<220>

<223> probe

<400> 40
agacaattag aatcagtgcc cct 23

<210> 41
<211> 23
<212> DNA
<213> Artificial

<220>
<223> probe

<400> 41
gcattgaggt attgttgctc cca 23

<210> 42
<211> 23
<212> DNA
<213> Artificial

<220>
<223> probe

<400> 42
cgagagtctg taatagccga tgc 23

<210> 43
<211> 23
<212> DNA
<213> Artificial

<220>
<223> probe

<400> 43
tgccgtgata cttaactacg cta 23

<210> 44
<211> 23
<212> DNA
<213> Artificial

<220>
<223> probe

<400> 44
gagtccgcaa aaatatagga ggc 23

<210> 45
<211> 23
<212> DNA
<213> Artificial

<220>
<223> probe

<400> 45

gcctcacata actggagaaa cct 23

<210> 46
<211> 23
<212> DNA
<213> Artificial

<220>
<223> probe

<400> 46
cgccaatgac aataagttga ggc 23

<210> 47
<211> 23
<212> DNA
<213> Artificial

<220>
<223> probe

<400> 47
cgcgatataa cattaaccga ggc 23

<210> 48
<211> 23
<212> DNA
<213> Artificial

<220>
<223> probe

<400> 48
cacgcttagt tcctacctta ggc 23

<210> 49
<211> 23
<212> DNA
<213> Artificial

<220>
<223> probe

<400> 49
cgcgtcgaat tacttaatca cca 23

<210> 50
<211> 23
<212> DNA
<213> Artificial

<220>
<223> probe

<400> 50
gggataggta ttatgctcca gcc 23

<210> 51

<211> 23
<212> DNA
<213> Artificial

<220>
<223> probe

<400> 51
cgccattata caacggttca tgc 23

<210> 52
<211> 23
<212> DNA
<213> Artificial

<220>
<223> probe

<400> 52
gcctatatga accaagccac tgc 23

<210> 53
<211> 23
<212> DNA
<213> Artificial

<220>
<223> probe

<400> 53
cgccgtcagt acttgtatag atg 23

<210> 54
<211> 23
<212> DNA
<213> Artificial

<220>
<223> probe

<400> 54
gtcggtatcg aaaaggtact gca 23

<210> 55
<211> 23
<212> DNA
<213> Artificial

<220>
<223> probe

<400> 55
aggcagttca acctatatct gcg 23

<210> 56
<211> 23
<212> DNA
<213> Artificial

<220>
<223> probe

<400> 56
ggtcgtaaca ttgagaggag acg 23

<210> 57
<211> 23
<212> DNA
<213> Artificial

<220>
<223> probe

<400> 57
ggcgatttat tgctaactgg cta 23

<210> 58
<211> 23
<212> DNA
<213> Artificial

<220>
<223> probe

<400> 58
gcactaccgc taactatacg cta 23

<210> 59
<211> 23
<212> DNA
<213> Artificial

<220>
<223> probe

<400> 59
ggctcgtagt actccttaca tgc 23

<210> 60
<211> 23
<212> DNA
<213> Artificial

<220>
<223> probe

<400> 60
ggctctacaa acttgtgtcc atg 23

<210> 61
<211> 23
<212> DNA
<213> Artificial

<220>
<223> probe

<400> 61
ggtggagtga atctcactag act 23

<210> 62
<211> 23
<212> DNA
<213> Artificial

<220>
<223> probe

<400> 62
ctagcacaat taatcaatcc gcc 23

<210> 63
<211> 23
<212> DNA
<213> Artificial

<220>
<223> probe

<400> 63
gcagctgaat tgctatgatc acc 23

<210> 64
<211> 23
<212> DNA
<213> Artificial

<220>
<223> probe

<400> 64
gcctatagtg tcgattgtcc tcg 23

<210> 65
<211> 23
<212> DNA
<213> Artificial

<220>
<223> probe

<400> 65
cgatcacgga ttaatgtcac ccc 23

<210> 66
<211> 23
<212> DNA
<213> Artificial

<220>
<223> probe

<400> 66
aagagattta acttgagctc gcc 23

<210> 67
<211> 23
<212> DNA
<213> Artificial

<220>
<223> probe

<400> 67
tttgttgttc gatatcaggc gtg 23

<210> 68
<211> 23
<212> DNA
<213> Artificial

<220>
<223> probe

<400> 68
gcccgggaat agattataac gca 23

<210> 69
<211> 23
<212> DNA
<213> Artificial

<220>
<223> probe

<400> 69
gcatttttag taatccgagc gcc 23

<210> 70
<211> 23
<212> DNA
<213> Artificial

<220>
<223> probe

<400> 70
catggataag ttttcaagct gcg 23

<210> 71
<211> 23
<212> DNA
<213> Artificial

<220>
<223> probe

<400> 71
gagacaggta aaccctcaga gca 23

<210> 72
<211> 23
<212> DNA

<213> Artificial

<220>
<223> probe

<400> 72
tagcacccgt taaaacggaa atg 23

<210> 73
<211> 23
<212> DNA
<213> Artificial

<220>
<223> probe

<400> 73
tatgtttagt tgttgaaccg gcg 23

<210> 74
<211> 23
<212> DNA
<213> Artificial

<220>
<223> probe

<400> 74
cgatcagctc tatttccctc cca 23

<210> 75
<211> 23
<212> DNA
<213> Artificial

<220>
<223> probe

<400> 75
agtcagttaa tcagacgtga gca 23

<210> 76
<211> 23
<212> DNA
<213> Artificial

<220>
<223> probe

<400> 76
tggcaataca ataacgtatc gcg 23

<210> 77
<211> 23
<212> DNA
<213> Artificial

<220>

<223> probe

<400> 77
cgcagtttgc aagaacgaac aaa 23

<210> 78
<211> 23
<212> DNA
<213> Artificial

<220>
<223> probe

<400> 78
cgcgataatt gatacctacg ggc 23

<210> 79
<211> 23
<212> DNA
<213> Artificial

<220>
<223> probe

<400> 79
ggggtgtgag agctttttag acg 23

<210> 80
<211> 23
<212> DNA
<213> Artificial

<220>
<223> probe

<400> 80
gggatccgta acaagtgtgt tag 23

<210> 81
<211> 23
<212> DNA
<213> Artificial

<220>
<223> probe

<400> 81
accactatga ttgaggaaac gcg 23

<210> 82
<211> 23
<212> DNA
<213> Artificial

<220>
<223> probe

<400> 82

cgtctttagt atcaaccctc cgc 23

<210> 83
<211> 23
<212> DNA
<213> Artificial

<220>
<223> probe

<400> 83
gcatacgaac ttctatatcg gcg 23

<210> 84
<211> 23
<212> DNA
<213> Artificial

<220>
<223> probe

<400> 84
ccgtgtgtat gagtatgaca gca 23

<210> 85
<211> 23
<212> DNA
<213> Artificial

<220>
<223> probe

<400> 85
tgctgtcttc gtgttttacc tag 23

<210> 86
<211> 23
<212> DNA
<213> Artificial

<220>
<223> probe

<400> 86
cgatcatgta aagctaactc gcg 23

<210> 87
<211> 23
<212> DNA
<213> Artificial

<220>
<223> probe

<400> 87
tgccgtcatt taaacgtaag ggt 23

<210> 88

<211> 23
<212> DNA
<213> Artificial

<220>
<223> probe

<400> 88
tggcaattac agttgttaac gca 23

<210> 89
<211> 23
<212> DNA
<213> Artificial

<220>
<223> probe

<400> 89
gagtcgaaga cctcctccta ctc 23

<210> 90
<211> 23
<212> DNA
<213> Artificial

<220>
<223> probe

<400> 90
atgccaatat gtactcgtga ctc 23

<210> 91
<211> 23
<212> DNA
<213> Artificial

<220>
<223> probe

<400> 91
gcatatagtg acggtaaggc gaa 23

<210> 92
<211> 23
<212> DNA
<213> Artificial

<220>
<223> probe

<400> 92
gcctcacttg taataagcgg gac 23

<210> 93
<211> 23
<212> DNA
<213> Artificial

<220>
<223> probe

<400> 93
gtcccaaaag cttcttacgg acg 23

<210> 94
<211> 23
<212> DNA
<213> Artificial

<220>
<223> probe

<400> 94
ctaggtacaa caccaactgt ctc 23

<210> 95
<211> 23
<212> DNA
<213> Artificial

<220>
<223> probe

<400> 95
tgccggttat acctttaagg acg 23

<210> 96
<211> 23
<212> DNA
<213> Artificial

<220>
<223> probe

<400> 96
ggctggttaa atgtaaatcc gcg 23

<210> 97
<211> 23
<212> DNA
<213> Artificial

<220>
<223> probe

<400> 97
cgcggtacta ttagaaaggg cta 23

<210> 98
<211> 23
<212> DNA
<213> Artificial

<220>
<223> probe

<400> 98
agtcgcttaa ttactccgga tgg 23

<210> 99
<211> 23
<212> DNA
<213> Artificial

<220>
<223> probe

<400> 99
cgctgttggt attaccttcc tcg 23

<210> 100
<211> 23
<212> DNA
<213> Artificial

<220>
<223> probe

<400> 100
tgcagtgtaa gcaactattg tct 23

## Description

TECHNICAL FIELD

The present Description relates to a method for detecting a target nucleic acid.

DESCRIPTION OF RELATED ART

Conventionally, DNA microarrays have been used to detect and identify species of oral microorganisms associated with oral disease for example (Health Labour Sciences Research Grant, Food Safety and Security Promotion Project: Studies Related to Evaluating the Effectiveness of So-called Health Foods (2006 Summary and Assigned Research Report)). Health Labour Sciences Research Grant, Food Safety and Security Promotion Project: Studies Related to Evaluating the Effectiveness of So-called Health Foods (2006 Summary and Assigned Research Report) describes a detection method using a DNA microarray with the following sequence of steps.

(1) A step of preparing a nucleic acid of a bacterium in a test sample.
(2) A step of preparing a labeled probe using the nucleic acid prepared in the nucleic acid preparation step as a template.
(3) A step of performing hybridization between the labeled probe and an oligonucleotide based on a sequence specific to the species or genus of the bacterium of interest.
(4) A step of determining whether or not hybridization has occurred in the hybridization step.

[Citation List]

[Non Patent Literature]

[Non Patent Literature 1] Health Labour Sciences Research Grant, Food Safety and Security Promotion Project: Studies Related to Evaluating the Effectiveness of So-called Health Foods (2006 Summary and Assigned Research Report)

SUMMARY

In the method described in Non Patent Literature 1, about 2 hours was required for reaction with the DNA microarray, or in other words for hybridization. Moreover, in some cases the result was that a labeled nucleic acid in the test sample bound not only to an oligonucleotide (probe) based on a specific sequence fixed to the DNA microarray, but also non-specifically to an oligonucleotide with a different sequence. In this case, it was sometimes necessary to reduce non-specific binding and the like by increasing the hybridization temperature. When this still did not solve the problem of non-specific hybridization, further array preparation and redesign of the probe sequences were sometimes necessary.
It is an object of this Description to provide a method for detecting a target nucleic acid, which is effective for reducing the time required in a hybridization step for detecting a target nucleic acid of a specific genome or the like and determining its sequence, and for reducing non-specific binding to a DNA microarray by a labeled nucleic acid obtained from a specific genome. It is another object of the Description to provide a method for detecting a target nucleic acid, which is also effective when a target nucleic acid supplied to hybridization is difficult to obtain specifically.
The inventors and others discovered that by using a DNA chip (DNA microarray) prepared by spotting tag sequences that do not require optimization of probe design or hybridization methods, it was possible to reduce non-specific binding of labeled nucleic acids to a DNA microarray using a specific DNA sample or RNA sample, and to obtain a strong signal with a short hybridization (incubation) time.
Specifically, the inventors set out to obtain a target nucleic acid (roughly tens of bp to 1 kbp in length) by amplifying a nucleic acid with a polymerase so as to include a probe design sequence region, from within the nucleic acid sequences of extracted genome DNA. A primer labeled with a fluorescent label or the like and a primer designed so as to include a low-homology region or the like of the genome DNA in the amplified region were used in this case. Next, the genome DNA amplification product labeled with the fluorescent label or the like and a tag probe, or in other words an oligonucleotide being a mediator provided with a first site that hybridizes specifically with the target nucleic acid and a second site that hybridizes specifically with a detection probe, were reacted with the detection probe on a DNA microarray consisting of the detection probe spotted and fixed on a glass substrate or the like. After completion of the reaction, this was washed and the fluorescent label or the like was measured, quantified and analyzed with a fluorescent scanner or the like.
It has been found that this method is effective for reducing the time required to determine the detection probe,

for reducing non-specific hybridization, and in cases in which it is difficult to obtain a specific target nucleic acid for hybridization. This Description provides the following means based on these findings.

The present invention is as set out in the claims.

BRIEF DESCRIPTION OF DRAWINGS

FIG. 1 shows an outline of one example of the detection method of the invention;
FIG. 2 shows an outline of another example of the detection method of the invention;
FIG. 3 shows an outline of one example of a conventional detection method;
FIG. 4 shows a fluorescent image obtained using a mediator I obtained in Example 1;
FIG. 5 shows a fluorescent image obtained using a mediator II obtained in Example 1;
FIG. 6 shows a comparison of fluorescent intensities obtained using a mediator I and a mediator II obtained in Example 1, with the left part showing the results for a microorganism A and the right part the results for a microorganism B;
FIG. 7 shows a fluorescent image obtained in a comparative example;
FIG. 8 shows an array used in Example 2;
FIG. 9 shows evaluation results in Example 2;
FIG. 10 shows an array used in Example 3; and
FIG. 11 shows evaluation results in Example 3.

DETAILED DESCRIPTION OF INVENTION

The disclosures of this Description relate to a method for detecting a target nucleic acid. The disclosures of this Description relate to the following method for detecting a target nucleic acid.

That is,

(1) a signaling target nucleic acid is prepared having a signal generating part,
(2) this signaling target nucleic acid is brought into contact with a detection probe on a solid-phase body and an oligonucleotide being a mediator provided with a first site that hybridizes specifically with the signaling target nucleic acid and a second site that hybridizes specifically with the detection probe, in such a way that a hybridization product of these can be formed, and
(3) data based on the signal generating part of the signaling target nucleic acid is obtained on the solid-phase body.

In this detection method, the signaling target nucleic acid is hybridized with the detection probe via the mediator. The mediator has respective sequences specific to the signaling target nucleic acid and the detection probe pre-fixed on the solid-phase carrier. Using a mediator, the detection probe does not need to be optimized for each signaling target nucleic acid, non-specific hybridization can be controlled or avoided, and a pre-designed detection probe can be used in common to achieve specific hybridization in a short amount of time.

Moreover, in this detection method hybridization is performed with a signaling target nucleic acid that is associated with a detection probe via an intervening mediator. Thus, the signaling target nucleic acid itself is not associated with the detection probe, and can be obtained by an amplification reaction or the like performed on genome DNA independent of the predetermined detection probe. Therefore, the signaling target nucleic acid can be obtained stably and efficiently. It is also possible to omit an (additional) amplification reaction on the signaling target nucleic acid for purposes of associating it with the detection probe.

Moreover, with the method of detecting a target nucleic acid disclosed in this Description it is also possible to avoid the difficulties involved in specifically obtaining a target nucleic acid provided with both a signal generating part and a tag part that hybridizes specifically with the detection probe. Conventionally, there have been cases in which the desired amount of the target nucleic acid has not been obtained or another nucleic acid has been amplified by mistake when the target sequence of the target nucleic acid was not highly specific relative to other target nucleic acids mixed with it.

By contrast, with the disclosures of this Description a signaling target nucleic acid can be obtained with an emphasis on amplification efficiency. The resulting target nucleic acid can then be associated specifically via a mediator with a detection probe on a solid-phase body to produce a hybridization product.

Although a detection example using a low-homology region of genome DNA is discussed in the above explanation, this is not a limitation. For example, this technique can also be applied to the detection of SNPs (single nucleotide polymorphisms) and deletion and insertion sites in genome DNA, and also to detection using low-homology regions of RNA and other expression genes.

This detection method may encompass embodiments such as the following for example.

In this Description, a "nucleic acid" may be any DNA or RNA including cDNA, genome DNA, synthetic DNA, mRNA, total RNA, hnRNA and synthetic RNA, as well as peptide nucleic acids, morpholino nucleic acids, methylphosphonate nucleic acids, S-oligo nucleic acids and other artificially synthesized nucleic acids. It may also be single stranded or double stranded.

In this Description, moreover, a "target nucleic acid" may be any nucleic acid having any sequence. Typical examples include nucleic acids that may contain nucleotide sequences (target sequences) that serve as genetic markers in humans or non-human animals for the occurrence, diagnosis, prognosis and choice of medication and treatment for constitutional and genetic diseases and cancer and the like. Examples of markers include SNPs and other polymorphisms and congenital and acquired mutations. Target nucleic acids also include nucleic acids derived from pathogenic bacteria, viruses and other microorganisms.

In this Description, a "target sequence" is a sequence consisting of one or two or more bases specific to the target nucleic acid that is the object of detection. For example, it may be a partial sequence with low homology among the target nucleic acids, or a sequence with low complementarity or homology with other nucleic acids that may be contained in the sample. The target sequence may be a sequence specific to the target nucleic acid. The target sequence like this may have been artificially modified.

For the target nucleic acid, the sample described below or a nucleic acid fraction thereof may be used as is, but it is desirable to use an amplification product obtained by amplifying all of multiple nucleic acids by a PCR amplification reaction, and preferably an amplification reaction using multiplex PCR.

In this Description, a target nucleic acid is prepared from a "sample". A "sample" is a sample that may contain a target nucleic acid. The sample may be any sample containing nucleic acids, such as cells, tissue, blood, urine, sputum or the like. A person skilled in the art can obtain a fraction containing nucleic acids from these various kinds of samples as necessary with reference to prior art.

In this Description, an array is a solid-phase body comprising detection probes fixed on a solid-phase carrier in any pattern and by any form of binding. The mode of hybridization applied to the array is also not particularly limited.

The additional features and disclosures given below are given to provide further improved methods of detecting target nucleic acids and the like, and may be used either separately from or in combination with other features or inventions.

The combinations of features and steps disclosed in the detailed explanations below are not essential for implementing the disclosures of the Description in the broadest sense, and are only described in order to explain typical specific examples of the disclosures of this Description in particular. Moreover, the various features of the typical specific examples given above and below need not be combined according to the described specific examples or in the given order when providing additional and useful embodiments of the disclosures of this Description.

(Method of detecting target nucleic acid)

The method of detecting a target nucleic acid disclosed in this Description comprises a step of preparing a solid-phase body provided with a detection probe having a predetermined nucleotide sequence, a step of preparing a signaling target nucleic acid having a signal generating part for detecting the target nucleic acid, a step of bringing the signaling target nucleic acid, the detection probe, and an oligonucleotide being a mediator having a first site that hybridizes specifically with the target nucleic acid and a second site that hybridizes specifically with a part having the predetermined nucleotide sequence of the detection probe into contact with one another so that a hybridization product of these can be formed, and a step of obtaining data based on the signal generating part of the signaling target nucleic acid on the solid-phase body.

(Solid-phase body preparation step)

In the solid-phase body preparation step, a solid-phase body is prepared with the detection probe fixed thereon. Two or more detection probes may also be fixed to the solid-phase body so that two or more target nucleic acids can be detected. Such a solid-phase body may be prepared in advance before implementing the detection method, or may be obtained commercially, or may be prepared each time the detection method is implemented.

The solid-phase body may be of glass, a natural or artificial polymer material, or ceramic, metal or the like, without any particular limitations. Other examples include so-called porous materials consisting primarily of polymers such as polyether sulfone, nitrocellulose, nylon, vinylidene polyfluoride and the like. Such porous carriers are especially desirable for nucleic acid chromatography in which a detection probe fixed on a solid-phase carrier is hybridized with an amplified fragment by affinity chromatography. In this Description, nucleic acid chromatography is chromatography using a porous solid phase body carrier capable of dispersing and moving a liquid (developing medium) by capillary action, in which a nucleic acid is moved by this liquid inside the solid phase body carrier, forms a hybridization product by specific base pairing with a previously-prepared probe in the solid phase body carrier, and is thereby captured by the

solid phase body carrier.

The form, size and thickness of the solid-phase body are also not limited, and may be selected according to the mode of use. For example, a plate form, optionally flexible film form, or a spherical or other globular form may be selected.

The mode of hybridization (discussed below) may be considered in designing the form of the carrier. For example, when hybridization is performed in a microtube such as the Eppendorf tube™, which is widely used in testing and research, the array regions on the carrier are preferably of a size and form that allow them to be immersed in the hybridization solution contained in the tube. Such a carrier typically has a plane area of 150 $mm^2$ or less, an aspect ratio of 1.5 to 20, and a thickness of 0.01 mm to 0.3 mm.

When hybridization is performed using the principles of affinity chromatography with a detection probe fixed on a porous solid phase body carrier, it is desirable that at least the end of the carrier be of a size (width) and form that allow it to be immersed in a hybridization solution supplied to a microtube such as the Eppendorf tube™, which is widely used in testing and research. More preferably, it is in a long thin form provided with a site that can be contained within this kind of tube from near the bottom to the top of the tube. A solid-phase carrier for nucleic acid chromatography is not necessarily configured as a single solid-phase carrier. Multiple solid-phase carriers may be linked together as long as the expansion medium can be moved by capillary action throughout. In this case, the overall shape may be a sheet shape, a fine bar shape or any other shape that allows the chromatography solution to expand and disperse by capillary action.

The fixing configuration (two-dimensional configuration on the solid-phase body) of the detection probe is also not particularly limited, as long as data based on the signal generating part can be obtained by a detection method suited to the type of signal generating part and the like. Thus, detection properties and the like suited to the method used to detect the product of hybridization with the probe are considered when determining the fixing configuration of the detection probe. For example, in some cases the signal generating part emits fluorescence, and data from the signal generating part is obtained as data on fluorescent intensity with a suitable fluorescence detector. In such cases, a two-dimensional configuration (such as circular or other dots) that makes it easy to obtain intensity data is selected. In other cases, the signal generating part produces color that is visible in the visual light range or the like. In this case, the visually distinguishable characteristics of color, shape, size and significance (letters, graphics, symbols, numbers and combinations of these) are selected to make it easier to obtain data visually and evaluate the data.

The method of the invention employs nucleic acid chromatography. The two-dimensional form of the fixed detection probe may be any configuration, such as dots, lines or another shape. The probe fixing regions are normally in the form of lines perpendicular to the expansion direction of the chromatography expansion medium, and multiple lines are provided at suitable intervals in the direction of expansion.

The detection probe preferably has the predetermined nucleotide sequence pre-associated with the target nucleic acid. The detection sequence in the detection probe may be base sequences of SEQ ID NO: 1 to SEQ ID NO: 100 or their complementary base sequences. These base sequences have the same base length and have a melting temperature (Tm) of 40°C or higher and 80°C or lower, more preferably 50°C or higher and 70°C or lower, thereby giving homogeneous hybridization results under the same hybridization conditions.

A temperature calculated by the GC% method, the Wallace method or a method conforming to Current Protocols in Molecular Biology (described in Shujunsha Co., Ltd., Bio Jikken Illustrated 3, Hontou ni fueru PCR, p. 25) or the like may be adopted as the melting temperature, but in the present invention it is preferably calculated by the Nearest-Neighbor method, which addresses both the range of melting temperature and the effect of nucleotide sequence concentration. The melting temperature according to the Nearest-Neighbor method can be easily obtained using Visual OMP software (Tomy Digital Biology Co., Ltd.) or software (OligoCalculator; http://www.ngrl.co.jp/tool/ngrl_tool.html) provided by Nihon Gene Research Laboratories, Inc. (http://www.ngrl.co.jp/) for example. The sequences of SEQ ID NO:1 to SEQ ID NO:100 are listed in descending order of melting temperature (0.1 M probe concentration, 50 mM $Na^+$ ions, 1.5 mM $Mg^+$ ions) as calculated with Visual OMP.

The detection sequence in the detection probe is called also as an orthonormalization sequence and is designed based on the calculations on a consecutive identical length, melting temperature prediction by the Nearest-Neighbor method, a Hamming distance, secondary structure prediction on DNA sequences having certain base lengths obtained from random numbers. The orthonormalization sequences are base sequences of nucleic acids which have homogeneous melting temperatures and thus are designed so as to have the melting temperatures in a constant range, which do not inhibit hybridization with the complementary sequences because nucleic acids are structured intramolecularly, and which do not stably hybridize with base sequences other than complementary base sequences. Sequences contained in one orthonormalization sequence group hardly react or do not react to sequences other than a desired combination or within their sequences. When orthonormalization sequences are amplified by PCR, the amount of the nucleic acids quantitatively amplified correspond to an initial amount of the nucleic acids having the orthonormalization sequences without influenced by a problem such as cross-hybridization as mentioned above. Such orthonormalization sequences are reviewed in H. Yoshida and A. Suyama, "Solution to 3-SAT by breadth first search", DIMACS Vol.54, 9-20 (2000)

and Japanese Patent Application No. 2003-108126. The orthonormalization sequences can be designed by using the methods described in these documents.

A specific nucleotide sequence preferably consists of 20 to 50 nucleotides or more preferably 20 to 25 nucleotides.

(Signaling target nucleic acid preparation step)

The signaling target nucleic acid comprises a target nucleic acid and a signal generating part. The target nucleic acid is as described above.

(Signal generating part)

A conventionally known signal generating part can be selected appropriately and used as the signal generating part. Examples include various dyes including fluorescent substances that emit a fluorescent signal when excited by themselves, as well as substances that emit various signals by enzymatic reactions and antigen-antibody reactions when combined with a second component. Typically, a fluorescent label such as Cy3, Alexa555, Cy5 or Alexa647 can be used. Other possibilities are detection of color produced by combining biotin and streptavidin-HPR and treating with an appropriate substrate or the like, and a chromatography technique using latex particles.

Typical examples of signal generating part include labels using fluorescence, radioactivity, enzymes (for example, peroxidase, alkaline phosphatase, etc.), phosphorescence, chemoluminescence, coloration and the like.

The label is preferably a luminescent substance or chromogenic substance that presents luminescence or coloration that is detectable with the naked eye. That is, it is preferably a substance that can produce a signal that is itself directly visible to the naked eye, without the need for another component. This makes the detection process easier and more rapid. Typical examples of such substances include various pigments, dyes and other colorants. Equivalent substances include gold, silver and other precious metals, and copper and various other metals and alloys, as well as organic compounds containing these metals (which may be complex compounds). Mica and other inorganic compounds are also equivalent to colorants.

Typical examples of such labels include various dyes and pigments and chemoluminescent substances including luminol, isoluminol, acridinium compounds, olefins, enol ethers, enamines, aryl vinyl ethers, dioxenes, aryl imidazole, lucigenin, luciferin and aequorin. Other examples include latex or other particles labeled with these labels. Other examples include colloids or sols including gold colloids or sols and silver colloids or sols. Metal particles, inorganic particles and the like are also possible.

As discussed above, part of the label may be provided with particles. The average particle diameter of latex or other particles constituting part of the label is not particularly limited, but the mean particle diameter may be 20 nm to 20 μm for example, and is typically 40 nm to 10 μm, or preferably 0.1 μm to 10 μm, or more preferably 0.1 μm to 5 μm, or still more preferably 0.15 μm to 2 μm. Depending on the pore diameter of the solid phase body carrier 210, 500 nm or less is desirable, 250 nm or less is more desirable, 100 nm or less is still more desirable, and 50 nm or less is most desirable. The lower limit is preferably 0.1 nm or more, or more preferably 1 nm or more. For example, 0.1 nm to 250 nm is more desirable, and 1 nm to 250 nm is still more desirable. 0.1 nm to 100 nm is yet more desirable, and 1 nm to 50 nm is most desirable.

Preferred are particles made of a water-insoluble polymer material that can be suspended in an aqueous solution. Examples include polyethylene, polystyrene, styrene-styrene sulfonate copolymer, acrylic acid polymers, methacrylic acid polymers, acrylonitrile polymers, acrylonitrile-butadiene-styrene, polyvinyl acetate-acrylate, polyvinyl pyrrolidone or vinyl chloride-acrylate. Latex particles of such polymers having surface carboxyl, amino or aldehyde groups or other active groups are also possible.

The signal generating part may also be provided with materials that can be colored primarily or secondarily. Namely, the part may be provided with a molecule or substance (hereunder sometimes called the label binding substance) capable of binding these so as to allow ultimate recognition by the label. Protein-protein interactions and interactions between proteins and low-molecular-weight compounds and the like may also be used for example. Examples include antibodies in antigen-antibody reactions, biotin in avidin (streptavidin)-biotin systems, digoxigenin in anti-digoxigenin (DIG)-digoxigenin (DIG) systems, and haptens such as FITC and the like in anti-FITC-FITC systems. In this case, the label ultimately used in detection is modified so that it also functions as a site for binding between the label binding substance and another molecule or substance (an antigen for example, such as streptavidin, anti-FITC or the like) that interacts with the label binding substance. When the amplification product has a label binding substance, a complex can be formed between the label binding substance of the amplification product and a label provided with a site that binds with the label binding substance, either during the hybridization step or before or after this step, and the amplification product can be detected by means of the label.

Such labels and label binding substances can be obtained commercially, and methods for manufacturing labels

and label binding substances and for labeling particles with labels and the like are well known, and these can be obtained by a person skilled in the art using appropriate known techniques. Moreover, binding between such labels or labeled particles or label binding substances and DNA and other oligonucleotides can be accomplished as necessary via amino groups and other functional groups, and these matters are well known in the field.

The signaling target nucleic acid may be obtained by known methods. A person skilled in the art can as necessary obtain a signaling target nucleic acid comprising a label, label-binding substance or other signal generating part added to a desired target nucleic acid.

In the method of the present invention,

the signaling target nucleic acid is obtained by DNA fragment amplification using a DNA polymerase on a nucleic acid sample that may contain a target nucleic acid. Typically, it is obtained by performing a nucleic acid amplification reaction on a target nucleic acid using a primer set that includes a primer already provided with a label, label-binding substance or other signal generating part. Alternatively, it is obtained by performing a nucleic acid amplification reaction on a target nucleic acid using an NTP already provided with a label or label-binding substance.

Various known methods may be applied to the nucleic acid amplification, but various kinds of PCR including PCR, multiplex PCR and the like are typical. When performing a nucleic acid amplification step, the solution composition, temperature control and the like can be set appropriately by a person skilled in the art.

(Hybridization step)

The hybridization step is a step of bringing a signaling target nucleic acid, a detection probe and a mediator into contact with one another so that a hybridization product of these can be formed.

(Mediator)

The mediator is an oligonucleotide provided with a first site that hybridizes specifically target nucleic acid and a second site that hybridizes specifically with a predetermined nucleotide sequence part of the detection probe.

(First site)

The first site is a site that hybridizes specifically with the amplified region of the signaling target nucleic acid. More particularly, it is a site that hybridizes specifically with a target sequence constituting at least part of single-stranded DNA having a signal generating part in the signaling target nucleic acid (when this is double-stranded). The first site is sufficiently complementary to hybridize specifically with the target sequence, and is preferably entirely complementary to the target sequence. The target sequence is determined appropriately based on the melting temperature and relationships with other target nucleic acids to be detected simultaneously. The length of the nucleotide sequence of the first site is not particularly limited, but is preferably at least 15 but no more than 60 nucleotides.

(Second site)

The second site hybridizes specifically with a part having the predetermined nucleotide sequence of the detection probe. The predetermined nucleotide sequence of the detection probe is as described previously. That is, it may be an artificial sequence that is pre-associated with the target nucleic acid but is unrelated to the target nucleic acid, such as an orthonormalized sequence for example. The second site is sufficiently complementary to this detection sequence in the detection probe to allow specific hybridization. Preferably, it is entirely complementary to this detection sequence. The second site is preferably 20 to 50 or more preferably 20 to 25 nucleotides in length.

The mediator is an oligonucleotide provided with such a first site and second site, and is typically DNA, or else may have artificial nucleotides that are equivalent to natural nucleotides or a backbone that can be substituted with a sugar-phosphate backbone. A person skilled in the art can adopt such artificial nucleotides or backbone as necessary.

(Linker site/Joining site)

The mediator may also be provided with a linker site joining the first site and the second site. By providing such a linker site, it is possible to maintain a physical distance between the first site and the second site, and ensure good hybridization of the first site with the target nucleic acid and the second site with the detection probe.

A known linker site used in DNA can be selected and used appropriately as the linker site. The linker site is a joining site capable of inhibiting or arresting a DNA polymerase reaction preferably. The joinng site of the invention has a structure that cannot serve as a template during DNA elongation by DNA polymerase. That is, this joining site does not contain natural bases or natural base derivatives (natural bases or the like) that pair with natural bases. By not

including such natural bases and the like, it is possible to prevent the site from being a template, and inhibit or prevent DNA strands from being elongated by DNA polymerase. Therefore, this joining site may consist solely of a simple skeletal strand having no natural bases or the like. That is, it may be a sugar-phosphate backbone or other known backbone used in artificial oligonucleotides. The DNA polymerase includes various known DNA polymerases. Typical examples include DNA polymerases used in various kinds of PCR and other nucleic acid amplification methods.

**As the** mediator is provided with such a joining site as a linker site, possible effects with regard to capture of the target nucleic acid due to a new sequence occurred in the vicinity of the joining site of the specific sequence to the target nucleic acid (the first site) and tag (the second site) can be avoided. Further, even when the second site hybridized with the complementary sequence attached to the solid phase body and there is a possibility of the decrease of the degree of freedom of the mediator, such a joining site can provide free rotation of C-C bond of the linker site. As a result, mediator with the joining site has an advantageous effect that the first site can easily hybridize and capture the target nucleic acid as compared to the case of those without the linker site

This joining site may also be a chain-like joining group containing a single-stranded structure with an element number of 2 to 40, adjoining a nucleotide via a phosphate diester bond. If the element number is 1 or less, the DNA polymerase reaction may not be completely inhibited or arrested, while if the element number is over 40, nucleotide solubility may be diminished. Considering the effect of inhibiting or arresting the DNA polymerase reaction, the element number of the chain-like joining group is preferably 2 to 36 or more preferably 3 to 16.

This joining site contains a single bond in order to facilitate rotation at the joining site, and examples of single bonds include carbon-carbon, carbon-oxygen, carbon-nitrogen and S-S single bonds. This joining site preferably consists primarily of this single bond. As long as the joining site contains a single bond an aromatic ring or cycloalkane may also be included in part of the site.

An optionally substituted alkylene chain or polyoxyalkylene chain with an element number of 2 to 40 is preferably included as this joining site. Such a chain-like joining structure is structurally simple and easy to introduce as a joining site.

One example of this joining site is the joining site represented by Formula (1) below:

$$5'\text{-O-}C_mH_{2m}\text{-O-}3' \qquad \text{Formula (1)}$$

(wherein 5' represents the oxygen atom of a phosphate diester bond at the 5' end, 3' represents the phosphorus atom of a phosphate diester bond at the 3' end, and m is an integer from 2 to 40).

In Formula (1), m is more preferably 2 to 36, or still more preferably 3 to 16. A substituent of H in Formula (1) is typically an alkyl group, alkoxy group, hydroxyl group or the like. The number of carbon atoms in an alkyl group or alkoxy group is preferably 1 to 8, or more preferably 1 to 4. When there are 2 or more substituents, they may be the same or different. Preferably there are no substituents.

Another example of a joining site is the joining site represented by Formula (2) below:

$$5'\text{-}(OC_nH_{2n})_l\text{-v}3' \qquad \text{Formula (2)}$$

(wherein 5' represents the oxygen atom of a phosphate diester bond at the 5' end, 3' represents the phosphorus atom of a phosphate diester bond at the 3' end, n is an integer from 2 to 4,1 is 2 or an integer greater than 2, and (n + 1) x 1 is 40 or an integer smaller than 40).

In Formula (2), (n + 1) x 1 is preferably 2 to 36, or more preferably 3 to 16. A substituent of H in Formula (2) may be similar to the substituent in Formula (1).

The chain-like site shown below is an example of this joining site.

[C1]

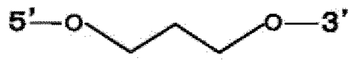

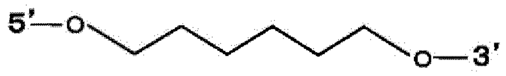

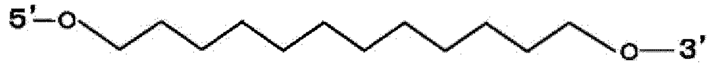

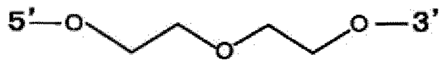

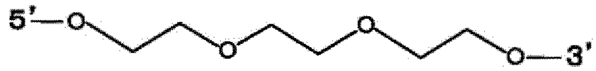

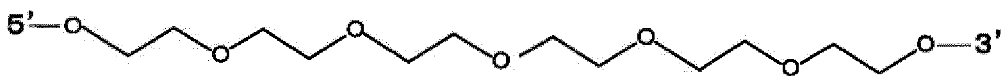

The chain-like site shown below is another example of this joining site.

[C2]

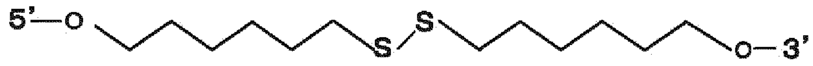

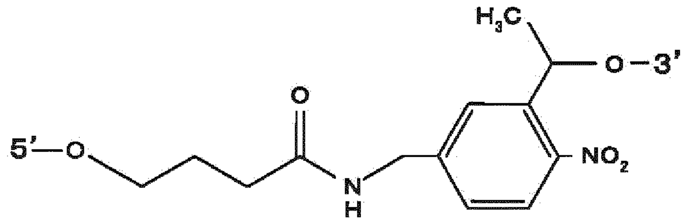

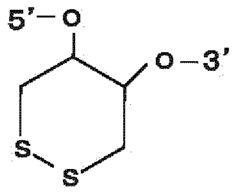

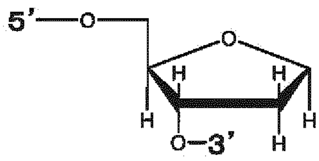

Other examples of the joining site include nucleic acid sequences having three-dimensional structures that inhibit the progress of the polymerase, such as strong hairpin structures and pseudoknot structures, as well as L-shaped nucleic acids, artificial nucleic acids and other target nucleic acid natural nucleic acids, and RNA, aliphatic chains and other non-nucleic acid structures. Examples of artificial nucleic acids include peptide nucleic acids, bridged nucleic acids, azobenzenes and the like.

Such a mediator and mediator including the joining site can be synthesized in accordance with ordinary oligonucleotide synthesis methods. For example, the joining site may be synthesized using a phosphoramidite reagent having an alkylene chain. Such reagents are well known, and can be obtained from GlenResearch or the like for example. Examples include the following reagent. In the formula below, DMT represents a typical dimethoxytrityl group as a hydroxyl protecting group, but this may also be another known hydroxyl protecting group. PA in the formula below represents a phosphoramidite group.

[C3]

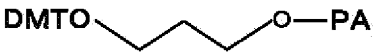

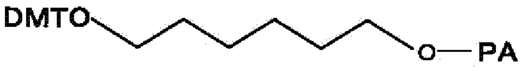

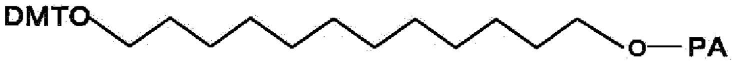

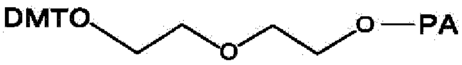

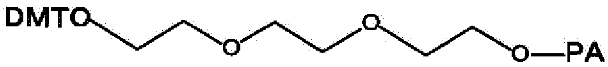

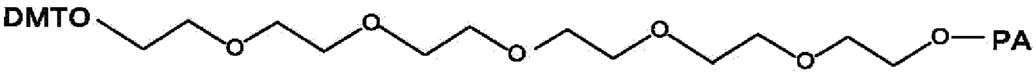

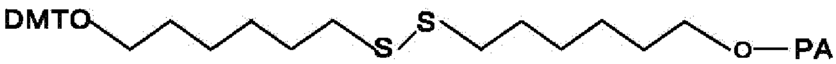

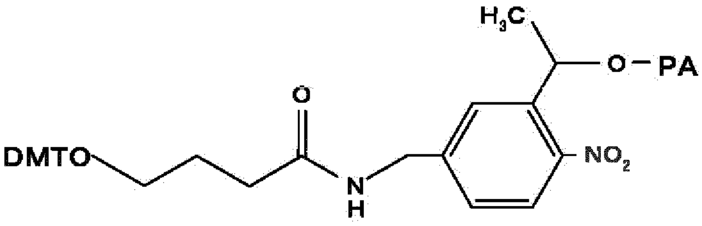

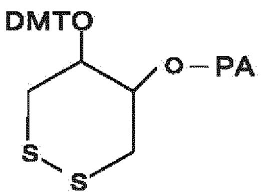

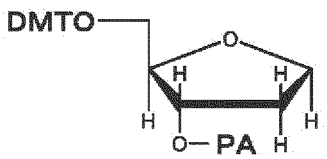

In the hybridization step, hybridization to form a hybridization product between the signaling target nucleic acid, the mediator and the detection probe can be performed by supplying the signaling target nucleic acid and the mediator to a solid-phase body with the detection probe fixed thereon. An ultimate object of hybridization is to form a hybridization product (composite) of the detection probe, the mediator, and single-stranded DNA of the target nucleic acid containing the signal generating part.

With such a hybridization step, hybridization between the detection probe and the mediator can be set in advance to be rapid and highly specific. By using hybridization between the mediator and the signaling target nucleic acid, moreover, it is possible to omit the operation or step of attaching a tag for specific hybridization between the signaling target nucleic acid and the detection probe. This means that the steps leading up to the hybridization step can be accomplished rapidly. It also means that the effort and time required for studying the operation or step of attaching

a tag to the signaling target nucleic acid can be eliminated.

To obtain this hybridization product, the signaling target nucleic acid and mediator can be hybridized specifically in advance, after which a primary product of the mediator and single-stranded DNA containing the signal generating part can be supplied to the solid-phase body to hybridize this primary product with the detection probe on the solid-phase body and obtain the hybridization product. The hybridization product can also be obtained by supplying in advance the mediator to the solid-phase body to form a separate primary product between the detection probe and the mediator, and then supplying the signaling target nucleic acid to this other primary composite and hybridizing them on the solid-phase body to obtain a hybridization product. Another method is to supply the signaling target nucleic and mediator simultaneously to the solid-phase body to obtain a hybridization product on the solid-phase body.

For the hybridization conditions, the temperature, time and medium can be selected appropriately by a person skilled in the art based on the kind of probe, the melting temperature and the like. Depending on the form of hybridization, temperature treatment can be performed as necessary prior to the hybridization step to convert the signaling target nucleic acid to a single strand or to form a composite with the mediator. A washing step may also be included after the hybridization step.

The method of the invention employs nucleic acid chromatography. The expansion medium is preferably an aqueous medium. The aqueous medium is not particularly limited, and may be water, an organic solvent soluble in water, or a mixture of water and 1 or 2 or more such organic solvents. Organic solvents soluble in water are well known to those skilled in the art, and examples include lower alcohols with 1 to 4 carbon atoms, DMSO, DMF, methyl acetate, ethyl acetate and other esters, and acetones and the like. The developing medium preferably consists primarily of water.

The embodiment of the nucleic acid chromatography is not particularly limited when performing the hybridization step. The aim may be to achieve development in a roughly horizontal state. In this case, chromatography is typically performed for example by dripping a fixed amount of the developing medium onto a liquid contact. The form of chromatography may also be designed to achieve roughly perpendicular development. In this case, chromatography is typically performed by holding the chromatography unit in a roughly perpendicular direction, and immersing the liquid contact part at the end of the unit in the developing medium.

Depending on the form of chromatography, the developing medium may disperse through the solid phase body carrier and expand to the probe region by the capillary phenomenon when the carrier is brought into contact with the developing medium and hybridization product is formed in the corresponding detection probe. There are no particular limitations on the conditions for the hybridization step, which may be performed in air at a temperature of 5°C to 40°C for example, or preferably 15°C to 35°C. Chromatography is initiated in the chromatography unit 100 for example by impregnating part of (the lower part or supply part) of a sheet-shaped chromatography unit with a width of 2.0 mm to 8.0 mm and a height (or length) of 20 mm to 100 mm with about 10 μl to 60 μl of developing medium. The time taken for the developing medium to completely pass through the probe regions 130 is about 2 to 50 minutes.

(Data-obtaining step)

The data-obtaining step is a step of obtaining based on the signal part of the signaling target nucleic acid on the solid-phase body and detecting a hybridization product between the amplified fragment and the detection probe.

The Data-obtaining step is a step in which signal strength data is obtained for the target nucleic acid based on the label retained on the hybridization product on the solid phase body after hybridization, to thereby detect the hybridization product. Signal strength data can be obtained by detecting a label signal from the label. Because the location of the detection probe associated with the target nucleic acid on the solid phase body is already known, it is possible to assess the presence or absence and proportion of the target nucleic acid by detecting the label signal.

To obtain signal strength data, conventional known methods can be selected and applied appropriately according to the form of the solid phase body and the type of label. Typically, oligonucleotides and the like that have not hybridized are first removed from the solid phase body by a washing operation or the like, and the fluorescent signal from the added label is then detected with an array scanner or the like, or a chemoluminescence reaction is performed on the label. Detection methods using flow cytometry are applicable when beads are used as the carrier.

In this Data-obtaining step, the presence or absence, proportion and the like of the target nucleic acid in the sample can be detected based on the signal strength data for the label. With this method, it is possible to reliably detect the target nucleic acid that is the object of detection even when detecting multiple target nucleic acids simultaneously.

The method of detecting a target nucleic acid explained above may also be used for detecting and identifying various kinds of microorganisms. For example, it can be used for academic detection and identification of microorganisms, as well as for detection and identification of microorganisms in clinical specimens. For example, it can be used to detect and identify oral microorganisms, including those associated with periodontitis.

[Examples]

The present invention is explained in detail below using examples, but the present invention is not limited by the following examples. In the examples below, all percentages are mass percentages.

[Example 1]

In this example, a hybridization product was formed with a detection probe on a solid-phase body using a signaling target nucleic acid and a mediator, the signal is detected, and the ability to detect the target nucleic acid was evaluated. In the examples given below, methods a method using mediator I and a method using mediator II are outlined in FIG. 1 and FIG. 2, respectively, and a method of a conventional example is outlined in FIG. 3.

(1) Preparation of DNA microarray

Aqueous solutions of dissolved synthetic oligo-DNAs (Nihon Gene Research Laboratories, Inc.) modified with amino groups at the 3'-ends were spotted as detection probes onto a Toyo Kohan Co., Ltd. Gene Slide glass substrate, using a Geneshot® spotter at NGK Insulators, Ltd. The synthetic oligo-DNA sequences used were the sequences D1_1 through D1_32 (SEQ ID NOS:1 to 32) and D1_100 (SEQ ID NO:100) out of the 100 sequences D1_1 through D1_100 (SEQ ID NOS:1 to 100) described in Supplementary Table 1 of the literature (Analytical Biochemistry 364 (2007) 78-85). After being spotted, this oligo-DNA was baked for one hour at 80°C. This was then washed for 15 minutes with 2 × SSC/0.2% SDS, washed for 5 minutes at 95°C with 2 × SSC/.0.2% SDS, and finally washed 3 times by shaking vertically 10 times with sterile water and centrifuged (1000 rpm × 3 min.) to remove the liquid.

(2) Amplification of genome DNA (target nucleic acid)

Of the microbial species of the oral cavity, the species used in evaluation were *Enterococcus faecaliso* (microorganism A) and *Pseudoramibacter alactolyticus* (microorganism B). The amplification primer sequences for amplifying the target nucleic acids of these microorganisms were as follows (unless otherwise specified, the nucleotide sequences are shown from the 5'-end to the 3'-end).

F-primer (common to microorganisms A and B):
Cy3- AGGTTAAAACTCAAAGGAATTGACG
R-primer (microorganism A):
GCAGATTCATTGGTCAGAGAACATATCTCTAGAGTGGT
R-primer (microorganism B):
CATCTAAAGCGTTCCCAGTTCCATATCTCTATTGCGCT

The conditions for the nucleic acid amplification reaction are as follows. That is, the reaction solution consisted of 15 μl of distilled water, 25 μl of Master Mix 1 (Multiplex PCR Kit QIAGEN), 3.75 μl of 10 pmol/μl F-primer, 3.75 μl of each 10 pmol/μl R-primer and 2.5 μl of template DNA, for a total of 50 μl. For the reaction conditions, the temperature was maintained for 15 minutes at 95°C, followed by 50 cycles of 0.5 minutes at 94°C, 0.5 minutes at 62°C and 0.5 minutes at 72°C, after which the temperature was maintained at 72°C for 10 minutes.

An amplification reaction was performed under these conditions, the amplified sample genes were purified with a QIAGEN Co. MinElute PCR Purification Kit, and amplification of the desired lengths was confirmed.

(3) Detection using DNA microarray

(2) The reaction on the DNA microarray and detection procedures using the fluorescent labeled amplification product obtained in (2) (signaling target nucleic acid) were as follows.

(Preparation of labeled sample solution for hybridization)

The labeled sample solution for hybridization consisted of 1.5 μl of control solution (concentration 2.5 nM), 9.0 μl of hybridization solution and 4.0 μl of mediator solution (concentration 100 nM) (subtotal 14.5 μl), with 4.0 μl of signaling target nucleic acid solution (PCR amplification product) added thereto for a total of 18.5 μl.

For the control solution, 10 μl of Alexa555-rD1_100 (100 nM) and 390 μl of TE (pH 8.0) (Alexa555-rD1_100 concentration 2.5 nM) were mixed for a total of 400 μl. The Alexa555-labeled oligo-DNA is DNA labeled with Alexa555 at the 5'-end of a sequence complementary to the D1_100 sequence out of the DNA microarray probes.

The hybridization solution was a mixture of 2.0 ml of 20 × SSC, 0.8 ml of 10% SDS, 12.0 ml of 100% formamide, 0.8 ml of 100 mM EDTA and 24.4 ml of milliQ water, for a total of 40.0 ml.

Two different mediator solutions (mediator I solution, mediator II solution) were prepared, containing 100 nM of each of 4 kinds of mediators I and II, respectively, using oligo-DNA of the following nucleotide sequences as the

mediators. 4 kinds of mediator I each provided with a first site and a second site were prepared, and 4 kinds of mediator II were prepared each provided with a first site, a second site and a joining site between the two. For the X in the nucleotide sequence of the mediators II (joining site (propylene chain)), DNA was synthesized using the phosphoramidite compound shown below.

[C4]

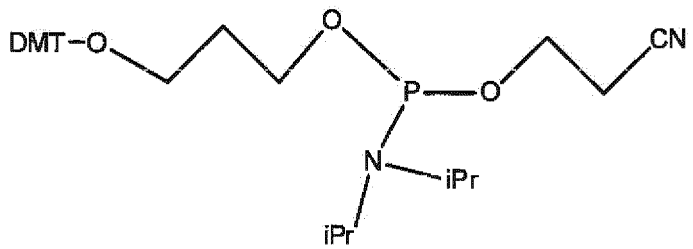

Mediator I

For microorganism A

Mediator I-A1: AGGGTAACCAACCAGCGAGCAGATTCATTGGTCAGAGAACA (corresponding to probe D1-001)
Mediator I-A2: AAAGTGCGTCTAAGTTCGCATCTAAAGCGTTCCCAGTTCCA (corresponding to probe D1-002)

For microorganism B

Mediator I-B1: CAGAGGGTAGCCAAGCCGGTCCGAAATTACAACGAAGCGAA (corresponding to probe D1-003)
Mediator I-B2: ATCCACATCATCCACCGAGTAGCGATTTCTTAGGATGCCT (corresponding to probe D1-004)

Mediator II

For microorganism A

Mediator II-AI: AGGGTAACCAACCAGCGAXGCAGATTCATTGGTCAGAGAACA (corresponding to probe D1-001)
Mediator II-A2: AAAGTGCGTCTAAGTTCGXCATCTAAAGCGTTCCCAGTTCCA (corresponding to probe D1-002)

For microorganism B

Mediator II-B1: CAGAGGGTAGCCAAGCCGXGTCCGAAATTACAACGAAGCGAA (corresponding to probe D1-003)
Mediator II-B2: ATCCACATCATCCACCGXAGTAGCGATTTCTTAGGATGCCT (corresponding to probe D1-004)

(Hybridization)

The prepared labeled sample solutions for hybridization were heated at 90°C for one minute with an Applied Biosystems GeneAmp PCR System 9700, and then heated at 80°C for 1 minute with a heat block (TAITEC DTU-N). 9 μl of each sample solution was applied to a spot area on a DNA microarray, and a hybridization reaction was performed by still standing for 30 minutes at 37°C, using a Comfort/Plus Thermoblock slide (Eppendorf) to prevent drying. The glass substrates after completion of the hybridization reaction were immersed in a glass staining vat containing a washing solution (188.0 ml MilliQ, 10.0 ml 20 × SSC, 2.0 ml 10% SDS (total 200.0 ml)), and shaken vertically for 5 minutes. The glass substrates were then transferred to a glass staining vat containing 1 × SSC, shaken vertically for 1 minute, and

dried centrifugally for 1 minute at 2,000 rpm to remove the moisture remaining on the glass substrate surfaces.

(Fluorescence detection by scanner)

Fluorescent images were obtained with a MolecularDevices GenePix 4000B, with the LaserPower and PMT adjusted appropriately. The results are shown in FIG. 4, FIG. 5 and FIG. 6.

As a comparative example, the following detection probes were modified at the 3'-ends with amino groups and spotted in a specific pattern on a Toyo Kohan Co., Ltd. glass substrate as in the example, a nucleic acid amplification reaction was performed as in the example on an amplification product amplified with the following primer set, which is common to microorganisms such as those detected in the example, and hybridization was performed on a glass substrate by 3 minutes of heating at 95°C, 3 minutes of cooling and 2 hours at 50°C using 10 μl of the resulting amplified product and 40 μl of the hybridization buffer used in the example (total 50 μl). Washing was performed using a shaker for 5 minutes at room temperature with 2 × SSC. Further washing was performed according to the example. As a result, fluorescent images were obtained with a scanner as in the example. The results are shown in FIG. 7.

Detection probe (microorganism A): ACCACTCTAGAGATA
Detection probe (microorganism B): AGCGCAATAGAGATA
F-primer (common to microorganisms A and B): Cy3-AGGTTAAAACTCAAAGGAATTGACG
R-primer (common to microorganisms A and B): Cy3-ATGGTGTGACGGGCGGTGTGT

As shown in FIGS. 4 to 6, hybridization using the mediators I and II was effective for both the microorganisms A and B, and the reactions were specific for all of the detection probes 1 to 4. The results for detection of the microorganisms A and B using the mediators I (without joining site) are shown at the left and right of FIG. 4, respectively. As shown in FIG. 4, the microorganisms A and B were detected satisfactorily using any of the mediators I. The results for detection of the microorganisms A and B using the mediators II (with joining sites) are shown at the left and right of FIG. 5, respectively. As shown in FIG. 5, the microorganisms A and B were detected satisfactorily using any of the mediators II.

It can be seen from the results above that hybridization with different detection probes can be accomplished with high specificity even using mediators that hybridize specifically with different target sequences of target nucleic acids amplified with the same primer set.

Moreover, the left part of FIG. 6 shows the results of a comparison of fluorescent intensity obtained for the same detection probe using the mediators I and II with the microorganism A. As shown in the left part of FIG. 6, the results for fluorescent intensity were stronger using the mediator II with any detection probe. The right part of FIG. 6 shows the results of a comparison of fluorescent intensity obtained for the same detection probe using the mediators I and II with the microorganism B. As shown in the right part of FIG. 6, the results for fluorescent intensity were stronger using the mediator II with any detection probe. It can be seen from these results that using the mediator II having a joining site, greater detection sensitivity is obtained with the joining site even in comparison to another mediator designed to hybridize with the same site of the target nucleic acid and to hybridize specifically with the same detection probe.

It was also shown that with the method of the example using a mediator and an artificial nucleotide sequence, highly specific hybridization could be achieved as described above, with a rapid hybridization time of 30 minutes at 37°C.

As shown in FIG. 7, moreover, microorganisms A and B could both be detected with specific probes even by the method of the comparative example (conventional method). In the conventional method, hybridization is performed at a higher temperature and for a longer period of time (50°C, 2 hours). Thus, the time required to detect the target nucleic acid is much longer than with the method of the example. Moreover, it can be seen that this is a higher-cost, lower-performance method overall than the method of the example because it requires the operations of preparing and fixing probes specific to the microorganisms A and B, respectively.

[Example 2]

In this example, a hybridization product was formed with a detection probe on a solid-phase body using a signaling target nucleic acid and a mediator as in Example 1, the signal was detected, and ability to detect the target nucleic acid was evaluated. The DNA microarray, genome DNA (target nucleic acid) amplification and detection using the DNA microarray were the same as in Example 1, except that the following primers were used in amplifying the genome DNA (target nucleic acid). As a comparative example, the same operations were performed as in the comparative example of Example 1 except that 10 μl of the amplification product and 10 μl of hybridization buffer (total 20 μl) were supplied to the DNA microchip as the hybridization solution.

F-primer (common to microorganisms A and B): Cy3-AGGTTAAAACTCAAAGGAATTGACG
R-primer (common to microorganisms A and B): Cy3-ATGGTGTGACGGGCGGTGTGT

As a result, as in Example 1, the microorganisms A and B were detected satisfactorily using any of the mediators I. Similarly, the microorganisms A and B were detected satisfactorily using any of the mediators II.

[Example 3]

In this example, a membrane-type DNA microarray was used to detect target nucleic acids.

(1) Preparation of membrane-type DNA microarray

Capture DNA probe solutions were spotted on Merck Millipore Hi-Flow Plus membrane plates (60 mm x 600 mm), using a NGK Insulators, Ltd. Geneshot® spotter with the discharge unit (inkjet method) described in Japanese Patent Application Publication No. 2003-75305 in the pattern and probes as shown in Fig. 8. For the synthetic oligo-DNA sequences, the 44 sequences out of the 100 sequences D1_1 to D1_100 described in the Supplementary **Table 1** of the literature (Analytical Biochemistry 364 (2007) 78-85) were used as common tags, as shown in Fig. 8. The numbers after D1 correspond to sequence ID numbers. For the synthetic oligonucleotides (DNA) used in spotting, DNA modified at the 5'-ends with proteins (albumin, avidin, etc.) was fixed.

(2) Amplification of sample genes

Evaluation was performed using the same microbial species as in Example 1 (*Enterococcus faecaliso* (microorganism A) and *Pseudoramibacter alactolyticus* (microorganism B)). The F primer and R primer shown below were used in common, and the amplification conditions were the same as in Example 1.

F-primer (common to microorganisms A and B): biotin-AGGTTAAAACTCAAAGGAATTGACG
R-primer (common to microorganisms A and B): biotin-ATGGTGTGACGGGCGGTGTGT

The amplified sample genes were purified with a QIAGEN MinElute PCR Purification Kit, and amplification of fragments of the desired length was confirmed

(3) Detection using DNA microarray

The reactions on the DNA microarray and the detection procedures were as follows using the samples amplified in (2). That is, the hybridization sample solution consisted of 200.0 μl of hybridization solution (0.5% Tween 20-1% BSA-PBS), 4.0 μl of mediator solution (concentration 100 nM) and 4.0 μl of control solution (concentration 2.5 nM) with 4.0 μl of each biotinylated target nucleic acid solution (PCR amplification product) added for a total of 212.0 μl. The control solution consisted of 10 μl of biotin-rD1_100 (100 nM) mixed with 390 μl of TE (pH 8.0) for a total of 400 μl (final concentration of Alexa555-rD1_100 = 2.5 nM). This biotin-labeled oligo-DNA consisted of a sequence complementary to the D1_100 sequence out of the DNA microarray probes, labeled at the 5'-end with biotin.

The mediator solutions were prepared so as to contain 100 nM each of two kinds and one kind of mediator, respectively, using oligo-DNA of the following nucleotide sequences as the mediators.

For microorganism A

Mediator II-A1: AGGGTAACCAACCAGCGAXGCAGATTCATTGGTCAGAGAACA (corresponding to probe D1-001)
Mediator II-A2: AAAGTGCGTCTAAGTTCGXCATCTAAAGCGTTCCCAGTTCCA (corresponding to probe D1-002)

For microorganism B

Mediator II-B1: CAGAGGGTAGCCAAGCCGXGTCCGAAATTACAACGAAGCGAA (corresponding to probe D1-003)

(3) Detection using DNA microarray

The DNA microarray was cut to a size that fit into a 0.2 ml tube and set in the tube, 200 μl of each of the hybridization solutions prepared above was added, heat denaturing was performed for 1 minute at a heat block temperature of 90°C, and a reaction was performed for 30 minutes at 37°C. After completion of the reaction, the DNA microarray was transferred to a 0.2 ml tube containing washing solution (0.1% Tween20-1 mM EDTA-TBS), and washed in a heat block at 37°C (37°C × 1 min, 37°C × 10 min, 37°C × 1 min).

The washed array was transferred to a 0.2 ml tube containing a mixed solution of biotin-HRP and streptavidin, and reacted for 20 minutes at room temperature. After completion of the reaction, the DNA microarray was transferred to a 0.2 ml tube containing washing solution (0.1% Tween 20-1 mM EDTA-TBS), and washed (room temperature × 1 min, room temperature × 10 min, room temperature × 1 min.). The washed microarray was then subjected to a coloring reaction for about 5 minutes using a Vector Laboratories TMB Peroxidase Substrate Kit, 3,3'5,5'-tetramethylbenzidine.

(Detection determination)

The coloring results on the array after drying are shown in FIG. 9. As shown in FIG. 9, strong coloring was observed when the coloring results were confirmed visually. These results show that a product of hybridization between a probe and a biotinylated target nucleic acid via a mediator can be detected on an array.

[Example 4]

In this example, a target method of acid was detected by method of acid chromatography.

Preparation of membrane-type DNA microarray(Solid phase body for chromatography)

Capture DNA probe solutions were spotted on Merck Millipore Hi-Flow Plus membrane plates (60 mm x 600 mm), using a NGK Insulators, Ltd. Geneshot® spotter with the discharge unit (inkjet method) described in Japanese Patent Application Publication No. 2003-75305 in the pattern and probes as shown in Fig. 10. For the synthetic oligo-DNA sequences, the 44 sequences out of the 100 sequences D1_1 to D1_100 described in the Supplementary **Table** 1 of the literature (Analytical Biochemistry 364 (2007) 78-85) were used as common tags, as shown in Fig. 10. The numbers after D1 correspond to sequence ID numbers. For the synthetic oligonucleotides (DNA) used in spotting, DNA modified at the 5'-ends with proteins (albumin, avidin, etc.) was fixed. After being spotted, this oligo-DNA was baked for one hour at 60°C.

(2) Amplification of sample genes

Evaluation was performed using the same microbial species as in Example 1 (*Enterococcus faecaliso* (microorganism A) and *Pseudoramibacter alactolyticus* (microorganism B)). The same F primer and R primer used in Example 2 were used in common as the primers, and the amplification conditions were the same as in Example 1. The amplified sample genes were purified with a QIAGEN MinElute PCR Purification Kit, after which amplification of fragments of the desired length was confirmed.

(3) Detection using solid-phase body for chromatography

A hybridization reaction and detection were performed by nucleic acid chromatography using the DNA amplified in (2).

First, 30.0 μl of PBS, 5.0 μl of mediator solution (concentration 100 nM) and 10.0 μl of biotinylated target nucleic acid solution (PCR amplification product) were added to a total of 45.0 μl to prepare an amplification reaction solution.

Using oligonucleotides of the following nucleotide sequences as mediators, the mediator solutions were prepared so as to contain 100 nM of each kind of mediator.

For microorganism A

Mediator II-A1: AGGGTAACCAACCAGCGAXGCAGATTCATTGGTCAGAGAACA (corresponding to probe D1-001)

For microorganism B

Mediator II-B1: CAGAGGGTAGCCAAGCCGXGTCCGAAATTACAACGAAGCGAA (corresponding to probe D1-003)

Next, 45 μl of the amplification reaction solution was heat denatured for 1 minute at a heat block temperature of 90°C, and cooled to prepare a reaction solution containing a product of hybridization between the mediator and the biotinylated target nucleic acid.

Following this reaction, 5 μl of latex solution was added to 45.0 μl of the reaction solution to obtain an expansion

solution, and this 50 μl was added to a 0.2 ml tube. The lower end of the DNA microchip, which had been cut so as to fit into the tube, was inserted into the 0.2 ml tube to initiate a hybridization reaction by chromatography. All of the expansion solution was taken up within about 20 minutes, completing the hybridization reaction by chromatography. For the latex solution, polystyrene latex beads containing a blue colorant were coated with avidin (streptavidin), and prepared with PBS to a specific concentration.

After completion of the reaction the array was dried, and the reaction sites were confirmed visually and photographed. The results are shown in FIG. 11. As shown in FIG. 11, a deep blue coloration was observed in the desired regions using the target nucleic acids obtained from microorganisms A and B. This shows that a hybridization product obtained via a mediator can be detected even in hybridization by chromatography.

[Sequence Table Free Text]

SEQ ID NOS:1 to 100: probes

## Claims

1. A method for detecting a target nucleic acid, the method comprising:

   a step of preparing a solid-phase body provided with a detection probe having a predetermined nucleotide sequence;
   a step of preparing a signaling target nucleic acid having a signal generating part for detecting the target nucleic acid;
   a step of bringing the signaling target nucleic acid into contact with the detection probe and an oligonucleotide being a mediator provided with a first site that hybridizes specifically with the target nucleic acid and a second site (preferably 20 to 50 nucleotides or more preferably 20 to 25 nucleotides in length) that hybridizes specifically with a part having the predetermined nucleotide sequence of the detection probe in such a way that a hybridization product thereof can be formed, wherein the step of bringing the signaling target nucleic acid into contact with the detection probe and the mediator is a step of using chromatography which uses the solid-phase body; and
   a step of obtaining data based on the signal generating part of the signaling target nucleic acid on the solid-phase body
   wherein the step of preparing the signaling target nucleic acid is a step of obtaining the signaling target nucleic acid by a DNA fragment amplification method using a DNA polymerase on a nucleic acid sample that may contain the target nucleic acid, and
   wherein the first site is a hybridization site that hybridizes specifically with the amplified region of the signaling target nucleic acid.

2. The method according to Claim 1, wherein the step of preparing the signaling target nucleic acid employs a DNA fragment amplification method using a DNA polymerase with a primer set including a primer having a signal generating part.

3. The method according to any of Claims 1 or 2, wherein the signal generating part contains a substance capable of primary or secondary coloration.

4. The method according to any one of Claims 1 to 3, wherein the detection probe has the predetermined nucleotide sequence pre-associated with the target nucleic acid.

**5.** The method according to Claim 4, wherein the predetermined nucleotide sequence of the detection probe is selected from orthonormalized sequences.

**6.** The method according to Claim 4 or 5, wherein the predetermined nucleotide sequence of the detection probe is selected from the nucleotide sequences represented by SEQ ID NOs:1 to 100, or the nucleotide sequences complementary to these nucleotide sequences.

**7.** The method according to any of Claims 1 to 6, wherein the step of preparing the solid-phase body is a step of preparing the solid-phase body provided with 2 or more of the detection probes capable of detecting 2 or more of the target nucleic acids.

**8.** The method according to any of Claims 1 to 7, wherein the mediator is also provided with a linker site joining the first site and the second site.

**9.** The method according to Claim 8, wherein the linker site of the mediator is a linker site capable of inhibiting or arresting a DNA polymerase reaction, and preferably includes an optionally substituted alkylene chain or polyoxyalkylene chain with an element number of 2 to 40 adjoining a nucleotide in the probe via a phosphate diester bond, and more preferably is represented by either of the formulae below:

$$5'\text{-O-}C_mH_{2m}\text{-O-}3' \qquad \text{Formula (1)}$$

(in the formula, 5' represents an oxygen atom of a phosphodiester bond at the 5' end, 3' represents a phosphorus atom of a phosphodiester bond at the 3' end, and m is an integer from 2 to 40); or

$$5'\text{-}(OC_nH_{2n})_1\text{-O-}3' \qquad \text{Formula (2)}$$

(in the formula, 5' represents an oxygen atom of a phosphodiester bond at the 5' end, 3' represents a phosphorus atom of a phosphodiester bond at the 3' end, n is an integer from 2 to 4, 1 is 2 or an integer greater than 2, and $(n + 1) \times 1$ is 40 or less.

**10.** The method according to any of Claims 1 to 9, wherein the step of bringing the signaling target nucleic acid into contact with the detection probe and the mediator is a step of forming a first hybridization product by specific hybridization of the amplified region and the first site, and a step of providing the first hybridization product to the solid-phase body and forming the hybridization product by specific hybridization of the second site and the detection probe.

**11.** The method according to any one of Claims 1 to 10, wherein in the chromatography step the chromatography is performed under temperature of 5°C to 40°C.

**Patentansprüche**

**1.** Verfahren zum Detektieren einer Zielnucleinsäure, wobei das Verfahren Folgendes umfasst:

einen Schritt des Herstellens eines Festphasenkörpers, der mit einer Detektionssonde mit einer vorbestimmten Nucleotidsequenz versehen ist;
einen Schritt des Herstellens einer signalisierenden Zielnucleinsäure mit einem signalerzeugenden Teil zum Detektieren der Zielnucleinsäure;
einen Schritt des In-Kontakt-Bringens der signalisierenden Zielnucleinsäure mit der Detektionssonde und einem Oligonucleotid, das ein Vermittler ist, der mit einer ersten Stelle, die spezifisch an die Zielnucleinsäure hybridisiert, und einer zweiten Stelle (mit einer Länge von vorzugsweise 20 bis 50 Nucleotiden oder mehr, noch bevorzugter mit einer Länge von 20 bis 25 Nucleotiden) versehen ist, die spezifisch an einen Teil mit der vorbestimmten Nucleotidsequenz der Detektionssonde hybridisiert, so dass ein Hybridisierungsprodukt davon gebildet werden kann, wobei der Schritt des In-Kontakt-Bringens der signalisierenden Zielnucleinsäure mit der Detektionssonde und dem Vermittler ein Schritt unter Verwendung einer Chromatographie ist, die den Festphasenkörper verwendet; und
einen Schritt des Erhaltens von Daten basierend auf dem signalerzeugenden Teil der signalisierenden Zielnucleinsäure auf dem Festphasenkörper,

wobei der Schritt des Herstellens der signalisierenden Zielnucleinsäure ein Schritt des Erhaltens der signalisierenden Zielnucleinsäure durch ein DNA-Fragment-Amplifizierungsverfahren unter Anwendung einer DNA-Polymerase auf eine Nucleinsäureprobe, die die Zielnucleinsäure enthalten kann, ist und
wobei die erste Stelle eine Hybridisierungsstelle ist, die spezifisch an die amplifizierte Region der signalisierenden Zielnucleinsäure hybridisiert.

**2.** Verfahren nach Anspruch 1, wobei der Schritt des Herstellens der signalisierenden Zielnucleinsäure ein DNA-Fragment-Amplifizierungsverfahren unter Verwendung einer DNA-Polymerase mit einem Primersatz, umfassend einen Primer mit einem signalerzeugenden Teil, verwendet.

**3.** Verfahren nach einem der Ansprüche 1 oder 2, wobei der signalerzeugende Teil eine Substanz enthält, die zu einer Primär- oder Sekundärfärbung in der Lage ist.

**4.** Verfahren nach einem der Ansprüche 1 bis 3, wobei die Detektionssonde die vorbestimmte Nucleotidsequenz in Vorassoziation mit der Zielnucleinsäure aufweist.

**5.** Verfahren nach Anspruch 4, wobei die vorbestimmte Nucleotidsequenz der Detektionssonde aus orthonormalisierten Spezies ausgewählt ist.

**6.** Verfahren nach Anspruch 4 oder 5, wobei die vorbestimmte Nucleotidsequenz der Detektionssonde aus den Nucleotidsequenzen, die durch Seq.-ID Nr. 1 bis 100 dargelegt sind, oder den Nucleotidsequenzen, die komplementär zu diesen Nucleotidsequenzen sind, ausgewählt werden.

**7.** Verfahren nach einem der Ansprüche 1 bis 6, wobei der Schritt des Herstellens des Festphasenkörpers ein Schritt der Herstellung des Festphasenkörpers ist, der mit 2 oder mehr der Detektionssonden versehen wurde, die dazu in der Lage sind, 2 oder mehr der Zielnucleinsäuren zu detektieren.

**8.** Verfahren nach einem der Ansprüche 1 bis 7, wobei der Vermittler ebenfalls mit einer Linkerstelle versehen ist, die die erste Stelle und die zweite Stelle verbindet.

**9.** Verfahren nach Anspruch 8, wobei die Linkerstelle des Vermittlers eine Linkerstelle ist, die dazu in der Lage ist, eine DNA-Polymerasereaktion zu inhibieren oder anzuhalten, und vorzugsweise eine gegebenenfalls substituierte Alkylenkette oder Polyoxyalkylenkette mit einer Elementzahl von 2 bis 40 umfasst, die über eine Phosphatdiesterbindung benachbart zu einem Nucleotid in der Sonde liegt, und noch bevorzugter durch eine der beiden nachstehenden Formeln dargestellt wird:

$$5'\text{-O-}C_mH_{2m}\text{-O-}3' \qquad \text{Formel (1)}$$

(in der Formel steht 5' für ein Sauerstoffatom einer Phosphodiesterbindung am 5'-Ende, 3' steht für ein Phosphoratom einer Phosphodiesterbindung am 3'-Ende, und m ist eine ganze Zahl von 2 bis 40); oder

$$5'\text{-}(OC_nH_{2n})_l\text{-O-}3' \qquad \text{Formel (2)}$$

(in der Formel steht 5' für ein Sauerstoffatom einer Phosphodiesterbindung am 5'-Ende, 3' steht für ein Phosphoratom einer Phosphodiesterbindung am 3'-Ende, n ist eine ganze Zahl von 2 bis 4, l = 2 oder eine ganze Zahl größer als 2, und (n+1) x l ist 40 oder weniger).

**10.** Verfahren nach einem der Ansprüche 1 bis 9, wobei der Schritt des In-Kontakt-Bringens der signalisierenden Zielnucleinsäure mit der Detektionssonde und dem Vermittler ein Schritt des Bildens eines ersten Hybridisierungsprodukts durch spezifische Hybridisierung der amplifizierten Region und der ersten Stelle und ein Schritt des Bereitstellens des ersten Hybridisierungsprodukts an dem Festphasenkörper und Bilden des Hybridisierungsprodukts durch spezifische Hybridisierung der zweiten Stelle und der Detektionssonde ist.

**11.** Verfahren nach einem der Ansprüche 1 bis 10, wobei die Chromatographie in dem Chromatographieschritt bei einer Temperator von 5 °C bis 40 °C durchgeführt wird.

## Revendications

1. Procédé de détection d'un acide nucléique cible, le procédé comprenant :

   une étape de préparation d'un corps en phase solide pourvu d'une sonde de détection ayant une séquence nucléotidique prédéterminée ;
   une étape de préparation d'un acide nucléique cible de signalisation comportant une partie de génération de signal pour détecter l'acide nucléique cible ;
   une étape de mise en contact de l'acide nucléique cible de signalisation avec la sonde de détection et un oligonucléotide qui est un médiateur pourvu d'un premier site qui s'hybride spécifiquement avec l'acide nucléique cible et d'un second site (d'une longueur de préférence de 20 à 50 nucléotides, ou de manière plus préférée de 20 à 25 nucléotides) qui s'hybride spécifiquement avec une partie ayant la séquence nucléotidique prédéterminée de la sonde de détection de telle manière qu'un produit d'hybridation de ceux-ci puisse être formé, dans lequel l'étape de mise en contact de l'acide nucléique cible de signalisation avec la sonde de détection et le médiateur est une étape d'utilisation d'une chromatographie qui utilise le corps en phase solide ; et
   une étape d'obtention de données sur la base de la partie de génération de signal de l'acide nucléique cible de signalisation sur le corps en phase solide
   dans lequel l'étape de préparation de l'acide nucléique cible de signalisation est une étape d'obtention de l'acide nucléique cible de signalisation par un procédé d'amplification de fragment d'ADN utilisant une ADN polymérase sur un échantillon d'acide nucléique qui peut contenir l'acide nucléique cible, et
   dans lequel le premier site est un site d'hybridation qui s'hybride spécifiquement avec la zone amplifiée de l'acide nucléique de signalisation.

2. Procédé selon la revendication 1, dans lequel l'étape de préparation de l'acide nucléique cible de signalisation utilise un procédé d'amplification de fragment d'ADN utilisant une ADN polymérase avec un ensemble d'amorces comprenant une amorce ayant un élément de génération de signal.

3. Procédé selon l'une quelconque des revendications 1 ou 2, dans lequel la partie de génération de signal contient une substance capable d'une coloration primaire ou secondaire.

4. Procédé selon l'une quelconque des revendications 1 à 3, dans lequel la sonde de détection a la séquence nucléotidique prédéterminée pré-associée à l'acide nucléique cible.

5. Procédé selon la revendication 4, dans lequel la séquence nucléotidique prédéterminée de la sonde de détection est choisie parmi des séquences orthonormalisées.

6. Procédé selon la revendication 4 ou 5, dans lequel la séquence nucléotidique prédéterminée de la sonde de détection est choisie parmi les séquences nucléotidiques représentées par SEQ ID NO : 1 à 100, ou les séquences nucléotidiques complémentaires de ces séquences nucléotidiques.

7. Procédé selon l'une quelconque des revendications 1 à 6, dans lequel l'étape de préparation du corps en phase solide est une étape de préparation du corps en phase solide muni de 2 ou plus des sondes de détection capables de détecter 2 ou plus de acides nucléiques cibles.

8. Procédé selon l'une quelconque des revendications 1 à 7, dans lequel le médiateur est également doté d'un site de liaison joignant le premier site et le second site.

9. Procédé selon la revendication 8, dans lequel le site de liaison du médiateur est un site de liaison capable d'inhiber ou d'arrêter une réaction d'ADN polymérase, et comprend de préférence une chaîne alkylène ou une chaîne polyoxyalkylène éventuellement substituée avec un nombre de 2 à 40 d'éléments adjacents à un nucléotide dans la sonde via une liaison phosphate diester, et de manière plus préférée est représenté par l'une ou l'autre des formules ci-dessous :

   $$5'\text{-O-}C_mH_{2m}\text{-O-}3' \qquad \text{Formule (1)}$$

   (dans la formule, 5' représente un atome d'oxygène d'une liaison phosphodiester à l'extrémité 5', 3' représente un atome de phosphore d'une liaison phosphodiester à l'extrémité 3', et m est un entier de 2 à 40) ; ou

$5'-(OC_nH2_n)_l-O-3'$ Formule (2)

(dans la formule, 5' représente un atome d'oxygène d'une liaison phosphodiester à l'extrémité 5', 3' représente un atome de phosphore d'une liaison phosphodiester à l'extrémité 3', n est un entier de 2 à 4, l vaut 2 ou un entier supérieur à 2 et (n+l) x l est égal ou inférieur à 40.

**10.** Procédé selon l'une quelconque des revendications 1 à 9, dans lequel l'étape de mise en contact de l'acide nucléique cible de signalisation avec la sonde de détection et le médiateur est une étape de formation d'un premier produit d'hybridation par hybridation spécifique de la zone amplifiée et du premier site, et une étape de fourniture du premier produit d'hybridation au corps en phase solide et de formation du produit d'hybridation par hybridation spécifique du second site et de la sonde de détection.

**11.** Procédé selon l'une quelconque des revendications 1 à 10, dans lequel dans l'étape de chromatographie, la chromatographie est effectuée à une température de 5°C à 40°C.

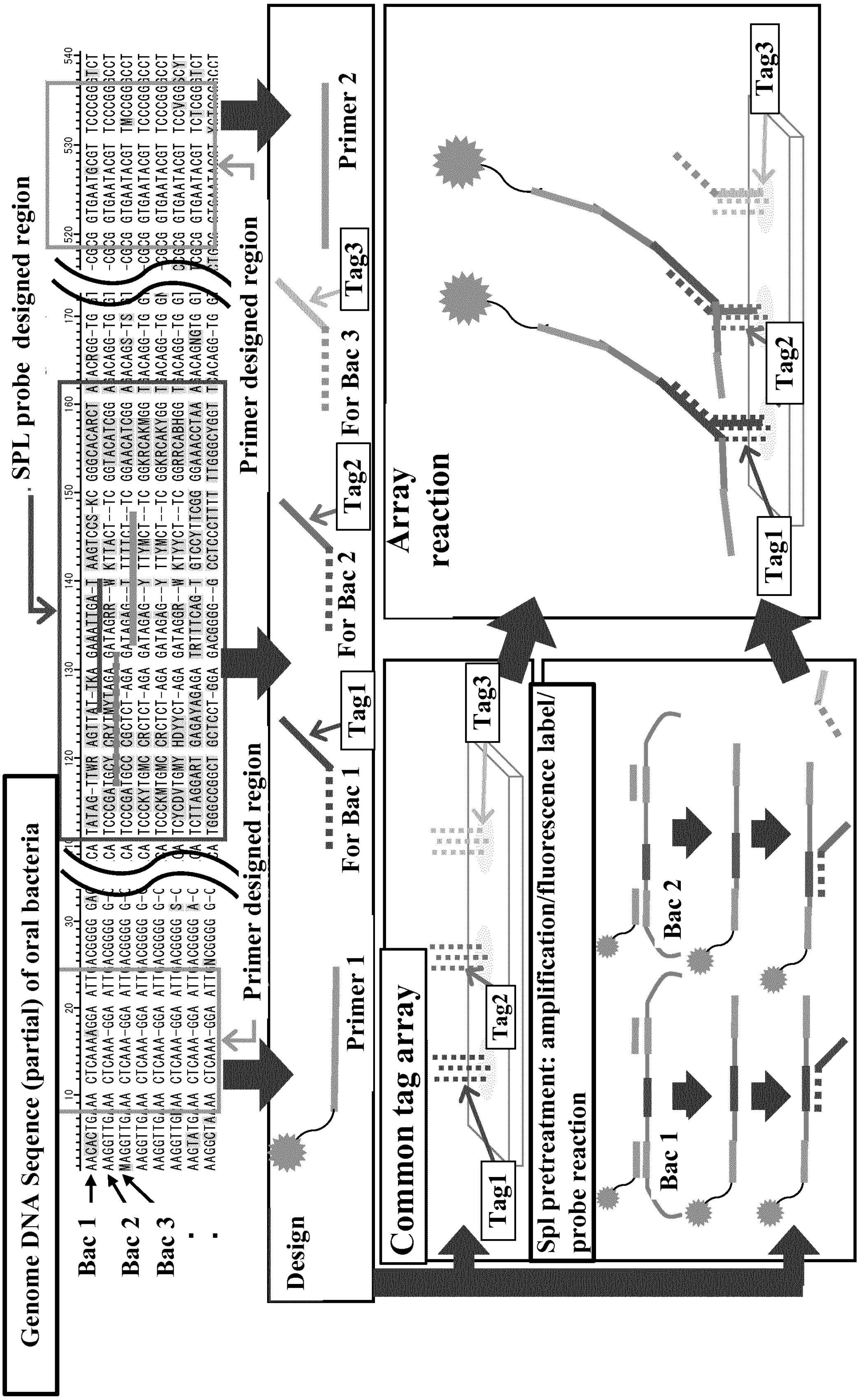
Genome DNA Seqence (partial) of oral bacteria
Bac 1
Bac 2
Bac 3
SPL probe designed region
Primer designed region
Primer designed region
Design
Primer 1
For Bac 1
Tag1
For Bac 2
Tag2
For Bac 3
Tag3
Primer 2
Common tag array
Tag1
Tag2
Tag3
Spl pretreatment: amplification/fluorescence label/ probe reaction
Bac 1
Bac 2
Array reaction
Tag1
Tag2
Tag3

Fig.1

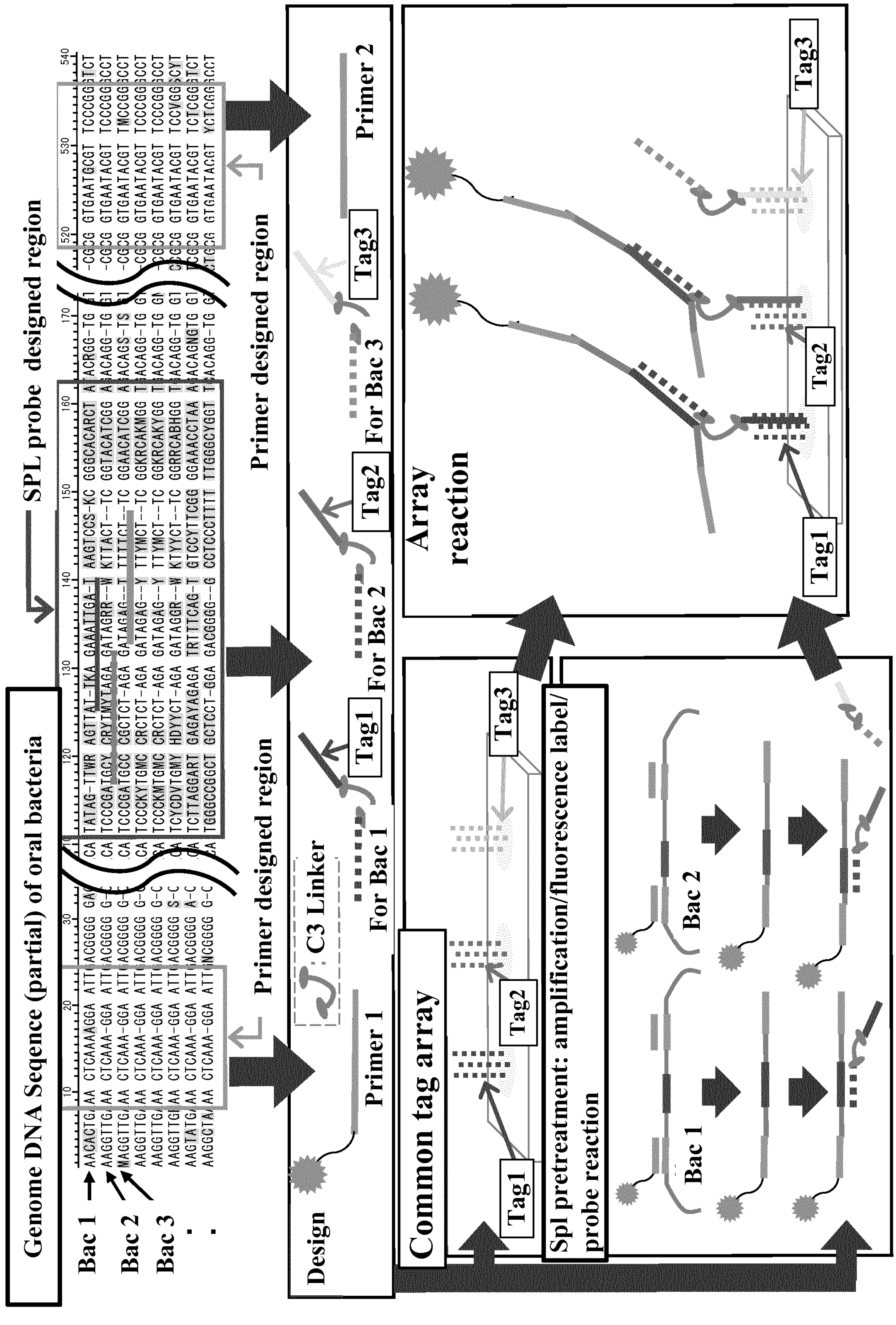
Genome DNA Seqence (partial) of oral bacteria
SPL probe designed region
Bac 1
Bac 2
Bac 3
Primer designed region
Primer designed region
Design
C3 Linker
Primer 1
Tag1
Tag2
Tag3
For Bac 1
For Bac 2
For Bac 3
Primer 2
Common tag array
Tag1
Tag2
Tag3
Spl pretreatment: amplification/fluorescence label/probe reaction
Bac 1
Bac 2
Array reaction
Tag1
Tag2
Tag3

Fig.2

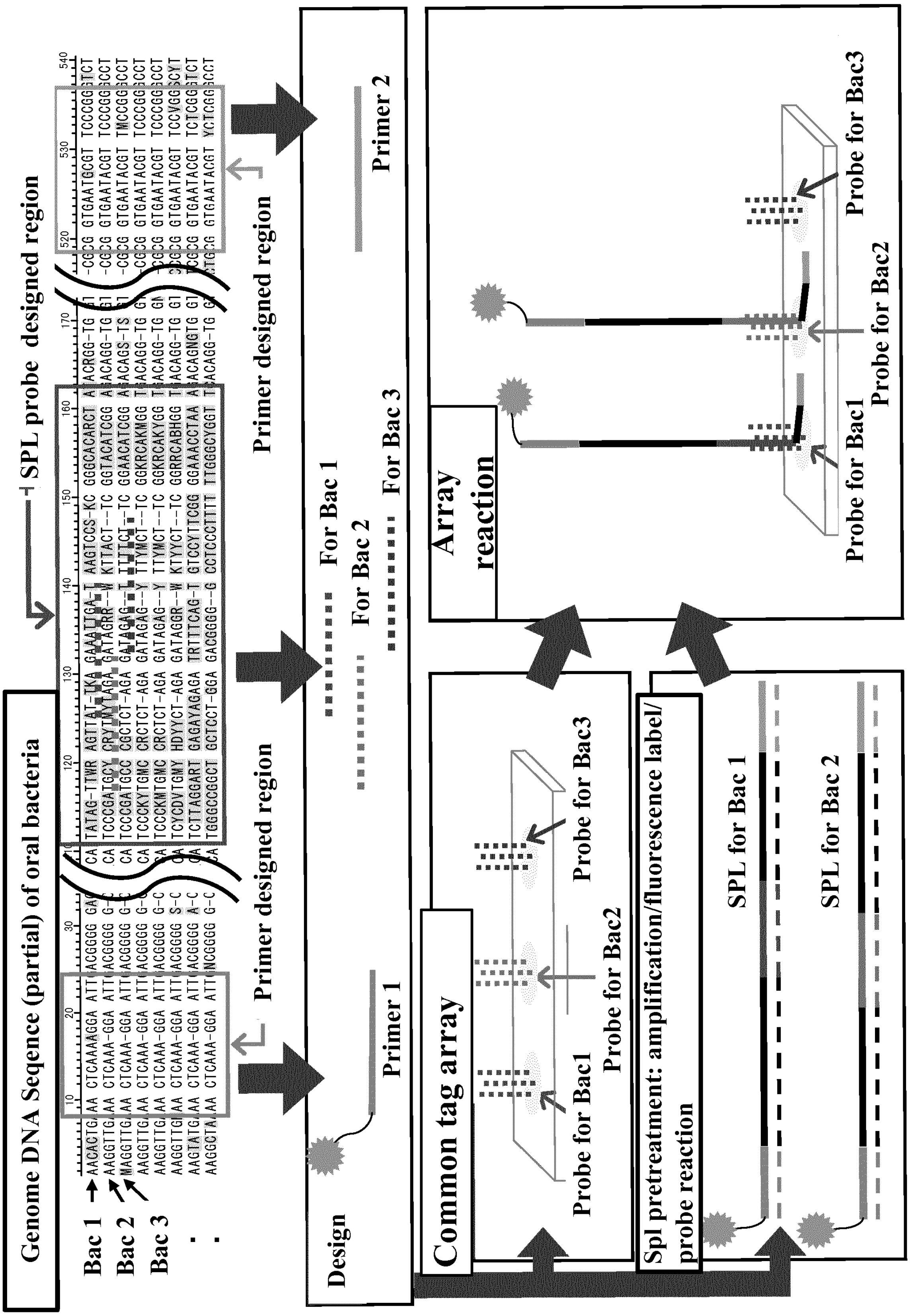
Genome DNA Seqence (partial) of oral bacteria
Bac 1
Bac 2
Bac 3
SPL probe designed region
Primer designed region
Primer designed region
Primer designed region
Design
Primer 1
For Bac 1
For Bac 2
For Bac 3
Primer 2
Common tag array
Probe for Bac1
Probe for Bac2
Probe for Bac3
Spl pretreatment: amplification/fluorescence label/probe reaction
SPL for Bac 1
SPL for Bac 2
Array reaction
Probe for Bac1
Probe for Bac2
Probe for Bac3

Fig.3

| Spl | BacteriaA | BacteriaB |
| --- | --- | --- |
| Result | 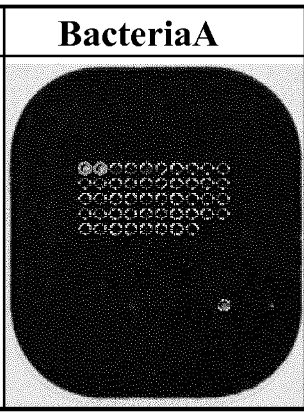 | 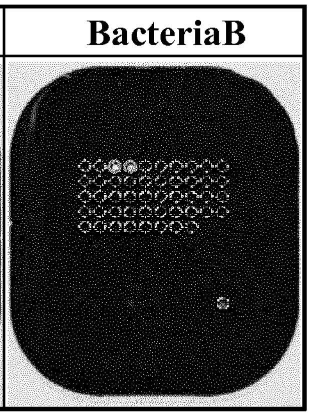 |

Fig.4

| Spl | BacteriaA | BacteriaB |
|---|---|---|
| Result | 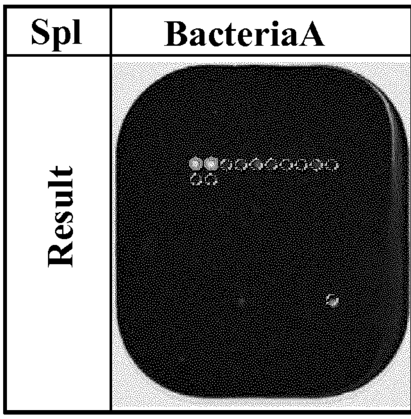 | 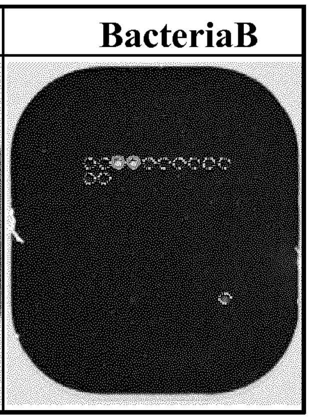 |

Fig.5

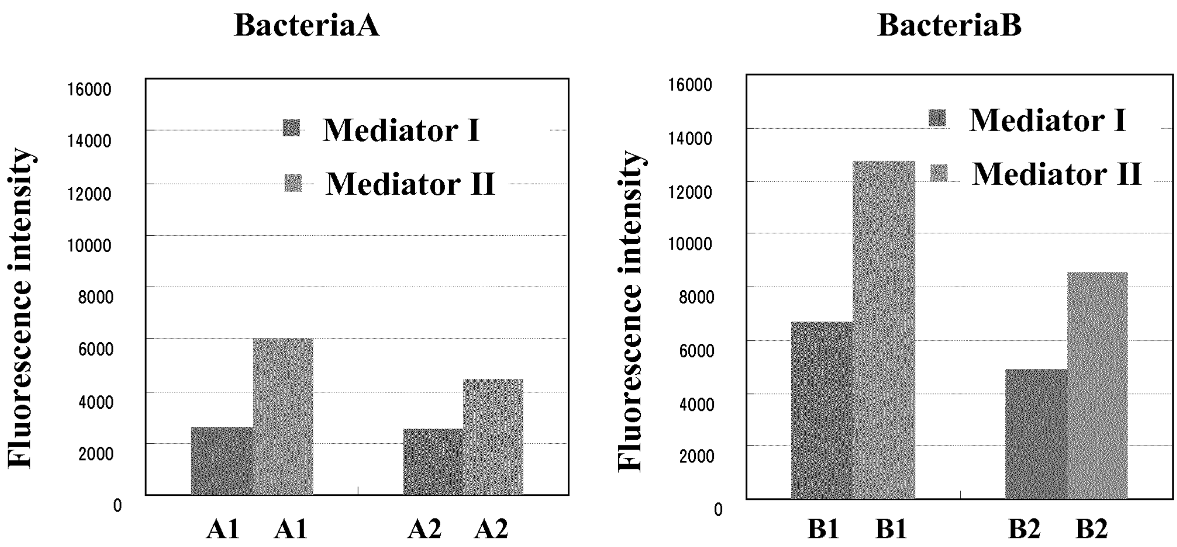
BacteriaA
BacteriaB
Fluorescence intensity
16000
14000
12000
10000
8000
6000
4000
2000
0
Mediator I
Mediator II
A1 A1 A2 A2
B1 B1 B2 B2

Fig.6

| No. | BacteriaA | BacteriaB |
| --- | --- | --- |
| Result | 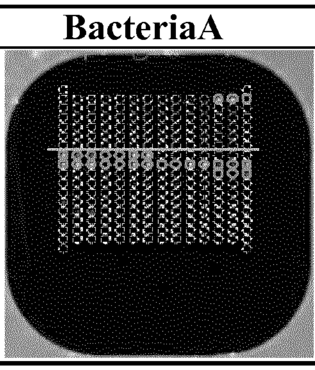 | 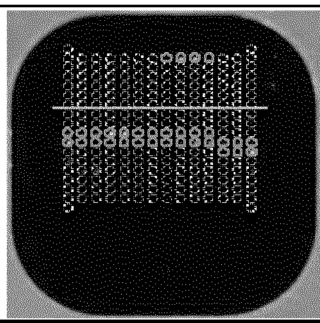 |

Fig.7

| | | | | |
|---|---|---|---|---|
| D1-100 | Coloring sample 1 | Marker (magenta) | Coloring sample 2 | D1-100 |
| D1-001 | D1-002 | D1-003 | D1-005 | D1-006 |
| D1-009 | D1-010 | D1-011 | D1-012 | D1-014 |
| D1-015 | D1-016 | D1-020 | D1-023 | D1-025 |
| D1-026 | D1-027 | D1-030 | D1-032 | D1-035 |
| D1-037 | D1-038 | D1-040 | D1-041 | D1-044 |
| D1-045 | D1-049 | D1-050 | D1-051 | D1-052 |
| D1-053 | D1-062 | D1-064 | D1-065 | D1-077 |
| D1-079 | D1-081 | D1-084 | D1-089 | D1-090 |
| D1-100 | D1-094 | D1-095 | D1-097 | D1-100 |

Fig.8

| Sample | BacteriaA | BacteriaB |
|---|---|---|
| Evalu-ation result | 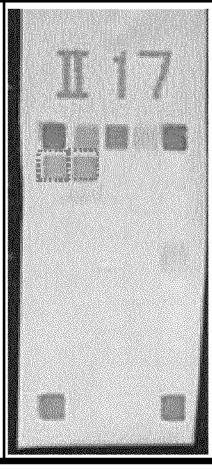  | 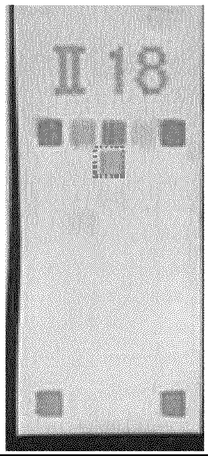  |

Hybridized with Probes D1-001 and 002

Hybridized with Probe D1-003

Fig.9

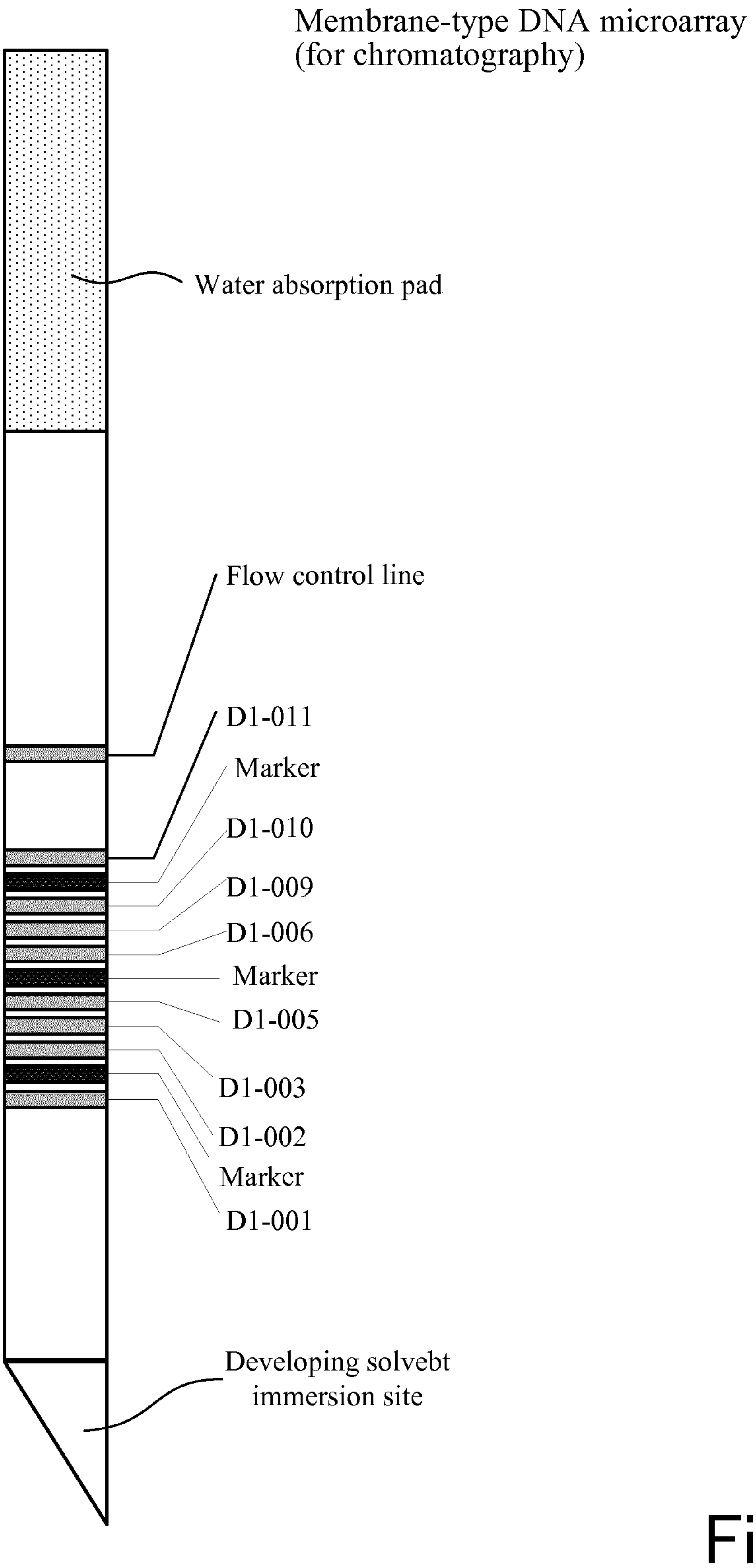
Membrane-type DNA microarray
(for chromatography)
Water absorption pad
Flow control line
D1-011
Marker
D1-010
D1-009
D1-006
Marker
D1-005
D1-003
D1-002
Marker
D1-001
Developing solvebt
immersion site

Fig.10

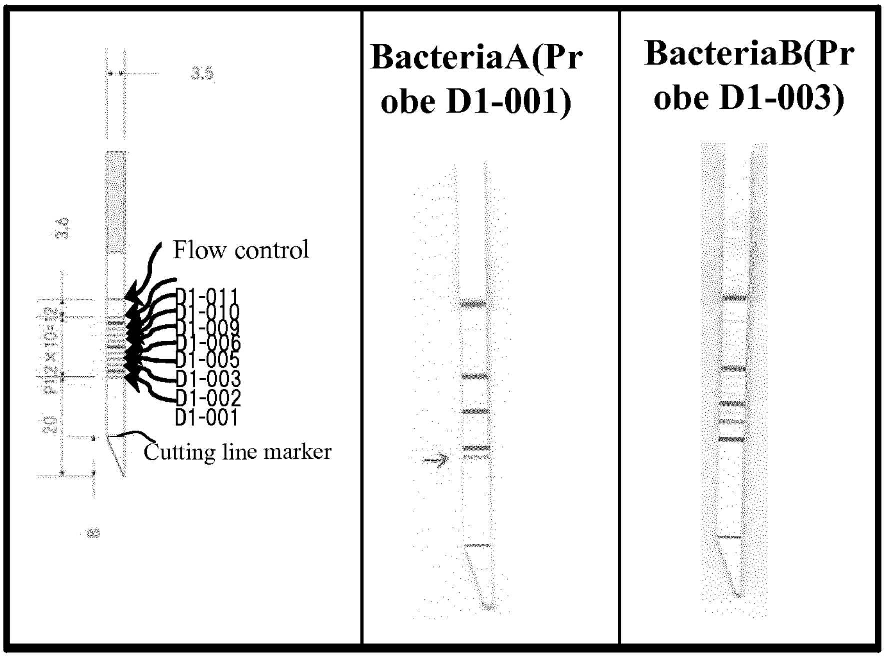
3.5
3.6
Flow control
D1-011
D1-010
D1-009
D1-006
D1-005
D1-003
D1-002
D1-001
20
Cutting line marker
8
BacteriaA(Probe D1-001)
BacteriaB(Probe D1-003)

Fig.11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2003108126 A
- JP 2003075305 A


### Non-patent literature cited in the description


- **H. YOSHIDA ; A. SUYAMA.** Solution to 3-SAT by breadth first search. *DIMACS,* 2000, vol. 54, 9-20
- *Analytical Biochemistry,* 2007, vol. 364, 78-85